(19)

**Europäisches Patentamt**
**European Patent Office**
**Office européen des brevets**

(11) **EP 2 029 266 B1**

(12) **EUROPEAN PATENT SPECIFICATION**

(45) Date of publication and mention
of the grant of the patent:
**20.04.2011 Bulletin 2011/16**

(51) Int Cl.:
*B01F 13/00* (2006.01)    *B01F 5/10* (2006.01)
*B01F 11/00* (2006.01)

(21) Application number: **07732799.7**

(86) International application number:
**PCT/GB2007/001775**

(22) Date of filing: **16.05.2007**

(87) International publication number:
**WO 2007/141474 (13.12.2007 Gazette 2007/50)**

(54) **MIXING APPARATUS AND METHOD OF DESIGNING A MIXING APPARATUS**

MISCHVORRICHTUNG UND VERFAHREN ZUR AUSLEGUNG EINER MISCHVORRICHTUNG

APPAREIL MÉLANGEUR ET PROCÉDÉ DESTINÉ À CONCEVOIR UN APPAREIL MÉLANGEUR

(84) Designated Contracting States:
**AT BE BG CH CY CZ DE DK EE ES FI FR GB GR
HU IE IS IT LI LT LU LV MC MT NL PL PT RO SE
SI SK TR**

(30) Priority: **05.06.2006 GB 0611041**

(43) Date of publication of application:
**04.03.2009 Bulletin 2009/10**

(73) Proprietor: **UNIVERSITY OF BRISTOL
Bristol, BS8 1TH (GB)**

(72) Inventors:
• **STURMAN, Robert, John
Bristol BS3 1AT (GB)**
• **WIGGINS, Stephen, Ray
Bristol BS8 4JU (GB)**

(74) Representative: **O'Connell, David Christopher
Haseltine Lake LLP
Redcliff Quay
120 Redcliff Street
Bristol BS1 6HU (GB)**

(56) References cited:
• **EVANS J ET AL: "PLANAR LAMINAR MIXER"
MEMS. PROCEEDINGS IEEE OF THE ANNUAL
INTERNATIONAL WORKSHOP ON MICRO
ELECTRO MECHANICAL SYSTEMS. AN
INVESTIGATION OF MICRO STRUCTURES,
SENSORS, ACTUATORS, MACHINES AND
ROBOTS, 26 January 1997 (1997-01-26), pages
96-101, XP000874342**
• **BARATUNDE AOLE COLA: "Optimization of a
pulsed source-sink microscale mixing device"
[Online] 1 December 2004 (2004-12-01),
VANDERBILT UNIVERSITY , NASHVILLE,
TENNESSEE , XP002446505 Retrieved from the
Internet: URL:http://etd.library.vanderbilt.edu/
ETD- db/available/etd-
12052004-230748/unrestric ted/
BarathesisFinial2.pdf> [retrieved on 2007-08-09]
the whole document**
• **BARATUNDE A. COLA; DAVID K. SCHAFFER;
TIMOTHY S. FISCHER; MARK A. STREMLER: "A
pulsed source-sink fluid mixing device"
JOURNAL OF MICROELECTROMECHANICAL
SYSTEMS, [Online] vol. 15, no. 1, 1 February 2006
(2006-02-01), pages 259-266, XP002446503
Retrieved from the Internet: URL:http://
ieeexplore.ieee.org/iel5/84/334 85/01588927.pdf?
tp=&isnumber=&arnumber=158 8927> cited in
the application**

**EP 2 029 266 B1**

**Description**

The present invention relates to techniques for inducing movement of particles of a substance in a chamber. More particularly, the present invention relates to an apparatus for inducing movement of particles in a chamber of said device and a method of designing the same. In particular, but not exclusively, the present invention relates to a microfluidic device and a method of designing a microfluidic device such as a DNA microarray.

The phenomenon of mixing arises in many technological and naturally occurring scenarios with length and time scales ranging from the very small (as in microfluidic applications), to the very large (mixing in the earth's oceans and atmosphere). The problem of mixing therefore presents itself in many forms including applications which seek to analyse, and potentially model, the mixing properties of a system, as well as applications which rely on controlling the mixing properties of a system. For example, applications which seek to achieve efficient mixing of two or more constituent fluids or, alternatively, to maintain segregation of those fluids, include food engineering, combustion, chemical reactors, bioreactors, microfluidic devices and polymer engineering. There are also technical applications which rely upon modelling or analysing mixing which occurs in nature, for example, the mixing in the earth's mantle or the dispersion of various substances present in the earth's atmosphere and oceans.

"Planar Laminar Mixer", Evans J et al, MEMS. Proceedings IEEE of the Annual International Workshop of Micro Electromechanical Systems, 26 January 1997 (XP000874342) discloses an approach to mixing for the MEMS environment based on chaotic advection. This document discloses an apparatus in accordance with the preamble of claim 1, and a method in accordance with the preamble of claim 15.

"Optimization of a pulsed source-sink microscale mixing device", Baratunde Aole Cola, Vanderbilt University, 1 December 2004 (XP002446505) discloses a mixing application using chaotic advection.

At the starting point of any mixing processes there exists two (or more) substances which occupy distinct domains. Usually, the process of mixing involves inducing movement of both of those substances in order to increase the interfacial area between them until reaching a final mixed state which comprises a homogeneous "mixture". The substances may differ, for example, in terms of their chemical composition or their temperature. Alternatively, the term "mixing" may refer to a situation where a first substance is substantially immobile within a domain, or chamber, whilst the particles of a second substance can move within the domain and thereby come into contact with the first substance. Usually, the objective of a technological mixing process is to reach a so-called mixed state in the minimum amount of time or using the least amount of energy.

One technological application which relies upon the movement of fluid particles is a DNA microarray. A DNA microarrray is constructed from DNA strands (the "probes") that are attached to an immobile surface, such as glass or silicon. The array is placed in a hybridization chamber containing a solution of mRNA (the "target") that is tagged with a fluorescent dye. Hybridization occurs when the target nucleic acid strand combines with a complementary probe nucleic acid strand according to base pairing rules. It can be detected by measuring the fluorescence of the probe spots. Movement of the target throughout the hybridization chamber is accomplished via two mixing mechanisms: diffusion ("static hybridization") and advection ("dynamic hybridization").

Ergodic theory is well established and provides a useful mathematical framework for quantifying the mixing properties of a system and for characterising a mixing process in a mathematical sense. Ergodic theory effectively ranks the mixing properties of a system from weakest, where a system is "ergodic", through a system which is "mixing", to a system which exhibits the "Bernoulli" property. Each of these terms have precise mathematical definitions. Ergodicity means that a system is indecomposable, in the sense that there are no non-trivial invariant regions, i.e., regions that do not mix with the surrounding fluid. Mixing means that any two regions of the fluid will become asymptotically independent of each other under evolution of the system. Bernoulli means that the motion of fluid particles is statistically indistinguishable from random coin tosses.

Ergodicity is the property that implies that a typical particle trajectory will visit all areas of the domain. A system having a chamber with a working region is said to be "ergodic" if an arbitrary particle comes arbitrarily close to every point in the working region. Thus, in the example of a DNA microarray, where hybridization is most efficient when each target nucleic acid can move throughout the solution and encounter every probe, it is desirable for the movement of the target to be ergodic.

"Mixing" is a stronger property and relates to the properties of two different substances of a system, the system can be mathematically described as "mixing" if the substances will mix together to create a homogeneous mixture. If a system is "mixing", it is implicit that it is also "ergodic".

There are a number of mechanisms by which particle motion can take place in order, for example, to mix two or more substances. If the substances are miscible, Brownian motion of individual fluid molecules, due to fluctuations in thermal energy, acts to homogenize the system at the molecular scale by means of molecular diffusion. However, in many scenarios, diffusion alone is a very inefficient means of mixing substances.

Mechanical mixing involves inducing motion of at least one of the substances, i.e. advection or convective motion, in order to create "flow". The dimensional qualities of a system, such as length and timescales, and the material parameters of the substances (e.g. molecular diffusivity, viscosity etc) will influence the characteristics of flow arising in a particular system. A laminar flow field, wherein the direction of velocity of the particles varies smoothly in space and time, gives rise to streamline flow wherein fluid tends to flow in parallel layers with no disruption between the layers. Turbulent flow arises when the velocity field of the particles is randomised as a result of mechanical forces acting on the system. The dimensionless Reynolds number, Re, is the ratio of inertial forces to viscous forces. If $U$ and $L$ denote characteristic velocity and length scales, $Re$ is $UL/V$, where v is the kinematical viscosity, which is the ratio of viscosity, $\mu$, and density, $\rho$, i.e, $v = \mu/\rho$. Small values of $Re$ correspond to viscous dominated, or laminar, flows, and large values of the Reynolds number correspond to turbulent flows. The spectrum of mixing problems which arise in nature and technology is wide, covering a range of Reynolds number and length scales, for example mixing in microfluidic devices; dispersion in oceans or the atmosphere; chemical reactors; combustion; mixing in physiological systems such as the lungs and blood vessels; blending of food additives in food engineering; polymer engineering; and geological mixing in the Earth's mantle.

The inefficiency of molecular diffusion as a mechanism for facilitating the motion of particles is particularly apparent at small length scales as illustrated by the following calculation. The typical distance L moved by a molecule with diffusion coefficient D in time t solely due to diffusion is given by: $L = \sqrt{Dt}$. Considering as an example the movement of a molecule by diffusion in a small channel or chamber, such as that comprised in a DNA microarray where diffusion coefficients typically range from between $10^{-5}$cm$^2$/s at the high end (corresponding to a small molecule) $10^{-7}$cm$^2$/s at the low end (typical of large molecules; e.g. haemoglobin in water corresponds to $10^{-7}$cm$^2$/s), it follows that a molecule moves only 1 to 3 mm in 24 hours. Since the typical horizontal length of a microarray is on the order of a few centimetres, it is clear that diffusion is not an efficient mechanism for mixing at small length scales. The problem is compounded by the fact that small length scale systems are resistive to turbulent flow. If flow can be made turbulent, then mixing follows naturally. However, in a small samples, particles will never overcome the inherent viscosity of the fluid and, even when subjected to convective forces, will continue to demonstrate a laminar flow field.

Techniques have therefore been developed which seek to accelerate the process of mixing in a system which exhibits a laminar flow field and which would otherwise be primarily dependent upon molecular diffusion to achieve a mixed state. One approach for increasing the efficiency of mixing in a laminar flow field is to stretch and fold the fluid layers and to thereby increase the inter-material area between the layers. Stretching of interfacial area has the effect of accelerating molecular diffusion since more interfacial area means more area for transfer. At the same time stretching diminishes striation thickness and therefore increases the concentration gradients and the mass flux.

Increasing the inter-material area between two substances, or between two layers of the same substance, can be visualised as the stretching of lines in 2D and, in 3D, the stretching of a surface. A fluid element of length δ(0) at time zero has length δ(t) at time t. The length stretch is defined as λ = δ(t)/δ(0). Thus, if mixing is effective, A increases nearly everywhere, though there can be regions of compression where λ< 1. In simple shear flow the fastest rate of stretching, dλ/dt, corresponds to when the element passes though the 45° orientation corresponding to the maximum direction of stretching in shear flow; for long times the stretching is linear in time, λ~t, as the element becomes aligned with the streamlines. In an elongational flow (e.g., a flow where the velocity field depends linearly on the spatial variables and contains a saddle type stagnation point) the rate of stretching is exponential, $\lambda \sim e^t$.

Based on the understanding that producing stretching and folding is a key factor to accelerating the mixing of two or more substances, or to facilitating the movement of particles of a substance within a chamber, the question of how best to achieve stretching and folding by advection is often critical to the design of numerous mixing systems. In the field of microfluidic devices, numerous techniques have been developed for achieving more efficient mixing by means of advection. For example, microfluidic devices are known which utilise air-driven bladders, or which provide means for enabling the boundaries of a mixing chamber to be move, for example by means of rotation of the hybridization chamber, by cavitation microstreaming, or by the use of magnetic stirring bars.

However, advection, i.e. inducing motion of the fluid, is often not sufficient to result in efficient mixing. In recent years it has been established that so-called *chaotic advection* provides a mechanism for enhancing the movement of particles within a domain and therefore for improving the mixing properties of a system, particularly microscale systems where turbulent fluid motion is not possible.

Mixer designs which utilise chaotic advection to increase the efficiency of the mixing process have been previously proposed in the context of DNA microarrays. These mixers comprise a fluid chamber, fluid supply means operable to allow fluid to be supplied to the chamber and fluid removal means operable to allow fluid to be removed from the chamber. Such designs, which involve cyclic removal and reinjection of the hybridization fluid, have been shown to generate a flow pattern which leads to efficient mixing.

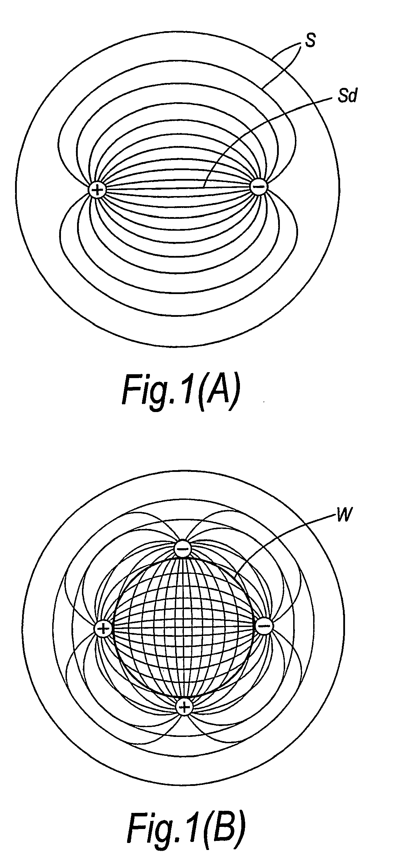

The process of removal from, and reinjection to, a chamber of particles of a substance can be modelled as a "source-sink" pair. As shown in Figure 1A, particles are shown to travel from the fluid supply means or "source" (+), to the fluid removal means, or "sink" (-), on a path called a "streamline" (S). The flow pattern arising from activation of a source-sink pair can be represented by a plurality of streamlines, wherein the most direct streamline ($S_d$) from a point source to a point sink generally represents the motion of particles travelling from source to sink with the greatest velocity, whilst particles which travel along one of the curved streamlines on either side of this shortest path travel more slowly. The positions that particles reach in a given time after a source-sink pair is activated defines a fluid front which will exhibit a peak along the streamline representing particles travelling with the greatest velocity. The derivative of the twist function generated by a source-sink pair therefore exhibits a turning point, or change in sign, along the peak in the fluid front. Hence, we can see that the twist function or "shear" breaks down along the fastest streamline between a source and the corresponding sink. Using the model of source-sink pairs, the streamlines generated by the removal and supply of a substance to a chamber can be computed analytically in order that the geometry of the flow pattern can be visualised and considered. The mathematical detail demonstrating how streamlines may be computed is given in the "theoretical analysis" section at the end of the description.

It has been previously shown that a sufficient condition for "chaos" is the crossing of streamlines. It is acknowledged that if flow-fields can be caused to intersect, such that the streamlines of one flow-field cross with the streamlines of another flow pattern so as to achieve cross-sectional flow, then chaos occurs amongst the particle trajectories. The present invention is founded on the understanding that "streamline crossing" provides a primary mechanism for creating chaotic advection.

The skill in designing mixers of the type which utilise the supply and removal of a substance to create a flow field, lies in determining the appropriate locations of the fluid supply means and the fluid removal means so as to generate a flow pattern in which the particle trajectories are chaotic. The pumping time is also relevant. However, determining the most appropriate locations for supply means and removal means of a fluidic system such as a DNA microarray has, conventionally, been a matter of trial and error.

A number of theoretical mixing protocols have been previously proposed which represent pulsed source-sink pairs and which specify the pumping times and the locations of source-sink pairs. For example, in a paper by Jones, S. W. & Aref, H. entitled "Chaotic advection in pulsed source-sink systems" published in Phys. Fluids, 31 (3), 469-485, a theoretical mixing protocol is proposed in which a source and a sink of equal strength are located in an unbounded domain. If both source and sink are switched on all the time then typical fluid particles travel along curved streamlines from source to sink. On arriving at the sink a particle is reinjected into the flow from the source according to a prescribed reinjection procedure. If the reinjection procedure is chosen so that a particle leaves the source along the same streamline as it entered the sink, then no chaotic motion occurs - there is no crossing of streamlines. In order to introduce streamline crossing, a time dependent feature is introduced to the system. The source and sink are switched on and off alternately. Then a typical particle path resembles a zig-zag pattern since the streamlines which are generated when the source is switched on lie in different directions to the streamlines which are generated when the sink is switched on.

An alternative mechanism for causing crossing of streamlines which has been previously proposed involves introducing a second source-sink pair into the system. In this situation the system is rendered time dependent by switching one source-sink pair on for a pumping time T1 whilst the second source-sink pair is switched off, and then switching the first source-sink pair off and the second pair on for a time T2. This produces two different streamline patterns which are effectively superimposed as the source-sink pairs are alternately switched on and off. Embodiments of the present invention are particularly concerned with the particle trajectories that arise from alternately generating first and second flow patterns in a domain or fluid chamber through action of a first, and then a second, source-sink pair. The resultant flow may be referred to as blinking flow and can effectively be considered to be the superposition of the two flow patterns. Each flow pattern, which comprises a plurality of streamlines, represents the paths that particles would follow from source to sink under the sole influence of each source sink pair. The idea of *blinking flow,* and variations on it, has been proposed, and implemented, in a number of micromixing devices.

An example of a previously considered mixing protocol which utilises blinking flow is discussed in a paper entitled "A pulsed source-sink fluid mixing device", by Cola, B. A., Schaffer, D. K., Fisher, T. S., & Stremler, M. A. (2006) and published in the Journal of Microelectromechanical Systems, 15(1), 259-265. According to the protocol described in this paper, two source-sink pairs are placed symmetrically in a circular boundary of radius 2 centred at the origin, with positions given by:

$$S_1 \quad = \quad \{S_+(-1,0), S_-(1,0)\}$$

$$S_2 \quad = \quad \{S_+(0,-1), S_-(0,1)\}$$

so that the flow acts first "left-to-right" under $S_1$ and then "bottom-to-top" up $S_2$. The streamlines generated for this protocol are shown in Figure 1B.

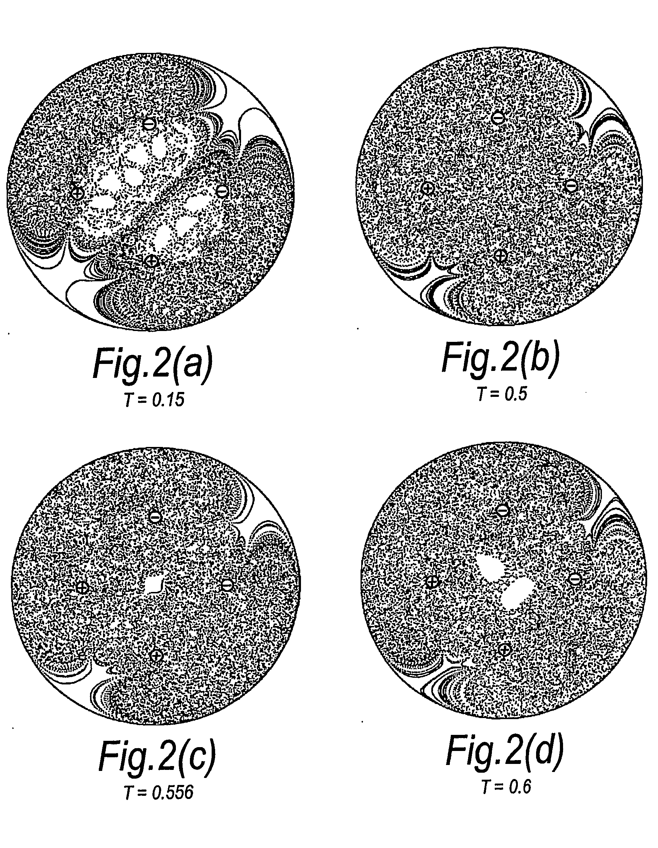

Using well established dynamical system theory, the present inventors have plotted a series of so-called Poincare sections, shown in Figure 2, which allow the motion of particles arising according to this protocol to be viewed stroboscopically at fixed discrete intervals of time. It can be seen from Figure 2, that when the pumping time T is fairly short (T = 0.15 in Figure 2(a))), several islands appear in a chaotic sea. A simplistic explanation for this is that for small T there is little stretching occurring. Increasing T (to T = 0.5 in Figure 2(b)) creates a flow which appears well-mixed. The cost of this improvement in mixing is the increased energy and time required to run the system for longer pumping times. Figure 2(c) shows a critical parameter, at which the pumping time is equal to the direct transfer time, T = 0.556 = TC. Here the centre point of the circle, which is on the direct transfer path for both S1 and S2, returns to the centre point under the action of both source-sink pairs, making the origin a fixed point of the Poincare map. A single island exists surrounding this point at the critical parameter. On increasing T, the central island increases in size, before splitting in two, forming a pair of islands, as in Figure 2(d), for T = 0.6. Increasing T further results in a well-mixed domain again, similar to Figure 2(a). The central island returns for $T = 2\tau_c$, and for $T = n\tau_c$ for all integers n. For each of these integers, the same pattern of the central island growing and then splitting into a pair of islands occurs.

Another example of a previously considered mixing protocol is discussed in a paper entitled "Study of a chaotic mixing system for DNA chip hybridization chambers" by Raynal et al. In this paper, a scheme is proposed in which the sources and sink take the form of syringes, so that fluid can be expelled from, or sucked into, the chamber. Thus, each syringe may act as either a source or sink (S$\pm$). The positions of the source-sink pairs may be defined as:

$$S_1 \quad = \quad \{S_+(-1,0), S_-(1,0)\}$$

$$S_2 \quad = \quad \{S_+(0,-1), S_-(0,1)\}$$

$$S_3 \quad = \quad \{S_+(1,0), S_-(-1,0)\}$$

$$S_4 \quad = \quad \{S_+(0,1), S_-(0,-1)\}$$

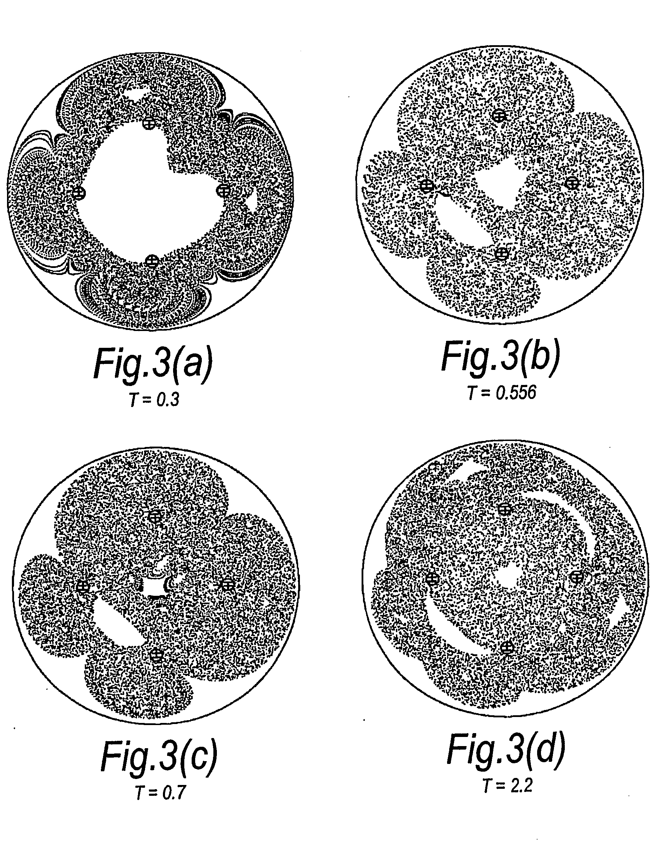

The flow generated by this scheme can be seen to go "left-to-right", "bottom-to-top", "right-to-left", and finally "top-to-bottom". The superimposed streamline pattern is shown in Figure 1b, with reversals in the direction of flow. Poincare sections, shown in Figure 3, have been constructed by the present inventors to illustrate the motion of particles for different pumping times under the scheme. Since each node acts as both sink and source, these are marked on the Poincare sections as a circled $\pm$.

Figure 3(a) is for pumping time T = 0.3. Here a large island is present near the centre of the circle which is likely to coincide with the working region of the chamber. It can be seen that simply increasing T does not appear to result in an ergodic flow. For all values of T up to the critical parameter T = TC = 0.556 islands appear, with the islands (including one central island) for T = TC = 0.556 shown in Figure 3b. Increasing T further has the effect of reducing the size of the central island, as in Figure 3(c) with T = 0.7. At this parameter value four new small islands now surround the central islands, and a further large island also persists. Furthermore, islands appear to persist even for large values of T (for example, Figure 3(d) for T = 2.2), although these reduce in size as T increases.

The present inventors have performed a careful analysis of a number of previously proposed protocols, including the ones discussed above, in order to evaluate their mixing properties. As illustrated by the Poincare sections shown in Figures 2 and 3, their analysis has revealed that whilst the use of pulsed source-sink pairs is an effective means for producing chaotic advection, the protocols still give rise to the presence of "islands" where chaotic particle trajectories are not expected.

Preferred embodiments of the present invention seek to provide an apparatus having a chamber which may be shown to demonstrate chaotic particle trajectories over the entire area of a predefined region, or a "working region", of the chamber. Thus, preferred embodiments of the present invention seek to provide a means by which the location and extent of chaotic regions (i.e. regions where the particle trajectories are chaotic) within a chamber can be controlled.

According to a first aspect of the present invention there is provided an apparatus in accordance with the features of claim 1.

Streamlines of the first and second flow pattern which cross each other rather than running parallel with each other, can be said to exhibit some degree of "transversality". Thus, it should be appreciated that streamlines which cross each other with a degree of transversality need not necessarily cross at ninety degrees. Physical boundary conditions mean that very close to the boundaries, streamlines become more and more aligned with the boundaries. Thus, in the case of a circular chamber, the streamlines get closer and closer to being circular as they approach the boundary. It is therefore inevitable that, at the boundary of a circular chamber, the streamlines of the first flow pattern will be substantially parallel to, or near to parallel to, the streamlines of the second flow pattern. In other words, due to physical constraints, it is impossible for streamlines to cross with a degree of transversality everywhere in a circular chamber. Thus, how far embodiments of the present invention can maximise the degree of transversality in the crossing of streamlines depends upon the shape of the chamber.

Embodiments of the present invention therefore seek to select the locations of the source-sink pairs (and thus the supply and removal means) so as to tend to maximise the transversality in the crossing of streamlines of said first and second flow patterns within a working region of the chamber whilst simultaneously tending to maximise the area over which the velocity function of said first and second flow patterns varies monotonically.

In most practical scenarios, these two objectives are essentially conflicting. For example, the degree of transversality in the crossing of streamlines will be a maximum when the fastest streamlines cross each other in the centre of the working region and at substantially ninety degrees. This can be achieved by locating the two source-sink pairs as shown in Figure 1B. However, the Poincare sections generated for the mixing protocol of Figure 1B, which are shown in Figure 3, indicate that the protocol is not that effective despite the high degree of transversality in the crossing of streamlines. Indeed, it can be seen from Figure 3 that islands of unmixed fluid prevail within the chamber no matter what the pumping time. One possible explanation for this observation proposed by the present inventors is that the change in the direction of the twist function, which takes place along the peak in the velocity profile of a flow pattern, actually gives rise to the islands of unmixed fluid that are revealed by the Poincare analysis. Thus, whilst the configuration shown in Figure 1B does maximise the degree of transversality in the crossing of streamlines within the working region, it also reduces the area over which the velocity function varies monotonically within the working region to the detriment of the resultant mixing properties. Embodiments of the present invention seek to provide an apparatus for inducing motion of particles of one or more substances, wherein the supply and removal means are located within the chamber such that when modelled as source-skin pairs, these two conflicting conditions are mutually maximised.

According to a preferred embodiment, there is provided an apparatus for inducing motion of particles of one or more substances, the apparatus comprising a chamber having a supply means, operable to supply a substance to the chamber, and a removal means, operable to remove a substance from the chamber, wherein said supply means and said removal means consists of three nodes which are operable to comprise first and second source-sink pairs, each source-sink pair comprising a source and a corresponding sink, wherein one said node is a common node, which common node comprises a source or a sink of said first source-sink pair and a source or a sink of said second source-sink pair,

wherein said nodes are positioned such that first and second shortest lines, which shortest lines represent the shortest distance between the common node and each of the other nodes, mutually define a working angle of between 60 and 120 degrees, wherein said nodes are positioned with respect to the chamber such that said working angle acts over the majority of the chamber including the central region thereof.

According to a preferred embodiment, there is provided an apparatus for inducing motion of particles of one or more substances, the apparatus comprising a chamber having a supply means, operable to supply a substance to the chamber, and a removal means, operable to remove a substance from the chamber, wherein said supply means and said removal means consists of three nodes which are operable to comprise first and second source-sink pairs, each source-sink pair comprising a source and a corresponding sink, wherein one said node is a common node, which common node comprises a source or a sink of said first source-sink pair and a source or a sink of said second source-sink pair, wherein said nodes are positioned such that first and second shortest lines, which shortest lines represent the shortest distance between the common node and each of the other nodes, mutually define a working angle of between 60 and 120 degrees, wherein all said nodes are positioned at, or near to, the outer region of the chamber.

It will be appreciated that whilst the common node may be implemented, in a practical sense, by means of a single supply/removal means, the provision of two separate supply/removal means positioned sufficiently close together as to act as a common node, is also envisaged within the context of embodiment of the present invention wherein the supply means and removal means consist of three nodes.

According to a preferred embodiment, there is provided an apparatus for inducing motion of particles of one or more substances, the apparatus comprising a chamber having a supply means, operable to supply a substance to the chamber, and a removal means, operable to remove a substance from the chamber, wherein said supply means and said removal means are further operable to comprise first and second source-sink pairs, each source-sink pair comprising a source and a corresponding sink, wherein said supply means and said removal means are configured such that a first shortest line between the source of the first source-sink pair and the corresponding sink is substantially orthogonal to a second shortest line between the source of the second source-sink pair and the corresponding sink, said first and second shortest lines representing the shortest distance between one said source and the corresponding sink, wherein said chamber is square or rectangular in shape and wherein said first and second shortest lines extend substantially alongside adjacent orthogonal edges of the chamber.

According to a preferred embodiment, there is provided an apparatus for inducing motion of particles of one or more substances, the apparatus comprising a chamber having a supply means, operable to supply a substance to the chamber, and a removal means, operable to remove a substance from the chamber, wherein said supply means and said removal means are further operable to comprise first and second source-sink pairs, each source-sink pair comprising a source and a corresponding sink, wherein said supply means and said removal means are configured such that a first shortest line between the source of the first source-sink pair and the corresponding sink defines a working angle of between 60 and 120 degrees with respect to a shortest line between the source of the second source-sink pair and the corresponding sink, said first and second shortest lines representing the shortest distance between the respect source and the corresponding sink, wherein said chamber is circular in shape and wherein said supply means and said removal means are configured such that the point of intersection, or the projected point of intersection, of said shortest lines is at or near the outer region of the chamber, and such that said working angle acts over the majority of the chamber including the central region thereof.

An advantage of these arrangements for both square/rectangular and circular chambers, is that whilst the degree of transversality will be high (a consequence of the angle between them), the shortest lines, which will represent the peak or turning point in the velocity profile of the flow pattern, do not extend cross the central region of the chamber. In this way, the working angle between the shortest lines, within which the velocity function will vary monotonically, will act over the majority of the chamber.

Preferably, according to any of the above aspects, the supply and removal means are configured such that the first shortest line is substantially orthogonal with respect to the second shortest line. Thus, in these circumstances, the working angle is approximately ninety degrees.

The working region (W) of the chamber is a predefined area of the chamber which in most practical scenarios generally lies in the centre of the chamber as shown in Figure1B. Preferably, according to particularly preferred embodiments, the working region may be defined as virtually the whole chamber. The supply means and/or the removal means may preferably be positioned outside the working region of the chamber.

Preferably, embodiments of the present invention alternately generate said first and second flow patterns in the fluid chamber by alternately operating the first source-sink pair and then the second source-sink pair. The time during which each source-sink pair is operated is called the pumping time T. The substance supplied by the supply means when acting as the source of the first source-sink pair may be the same as the substance supplied by the supply means when acting as the source of the second source-sink pair. Alternatively, the first and second source-sink pairs may be operable to supply and remove (and thereby induce motion of) two different substances within the chamber. The resultant blinking flow can effectively be considered to be the superposition of the two flow patterns as shown in Figure 1B. Each flow pattern can be illustrated as a plurality of streamlines, each streamline representing a possible path that particles can follow from source to sink under the influence of the respective source-sink pair. Thus, an apparatus embodying the present invention or designed according to an embodiment of the present invention, preferably comprises control means operable such that said supply means and said removal means are operable to alternately represent said first and second source-sink pairs.

The term monotonic means that the order, or direction, of a given function is preserved. Thus, in the context of the present invention, the direction of a twist function or "shear" acting on a fluid, which gives rise to a particular velocity profile, is said to be monotonic when it acts in the same direction.

In many practical scenarios, the supply means and the removal means may be modelled by a point source and a point sink respectively. For example, a supply means which comprises a syringe for injecting fluid to the chamber may be modelled as a point source. The flow pattern generated by supply and removal means which may be respectively modelled as a point source or sink, inherently exhibits a velocity profile which is non-monotonic, i.e. the velocity profile exhibits a peak along which the direction of the shear acting on the fluid changes. In such circumstances, in order to meet the conditions of the present invention, the supply means and the removal means are preferably positioned such that the streamline of the first flow pattern which represents the particles travelling with the greatest velocity (i.e. those particles which take the shortest time to travel from source to sink), does not cross the streamline of the second flow pattern which represents the particles travelling with the greatest velocity. In the case of a supply means and removal means which can be represented by point source/sinks, the streamline which represents the motion of particles which take the shortest time to travel from source to sink is generally the shortest streamline (in length). By ensuring that the "shortest" streamline of said first flow pattern does not cross the "shortest" streamline of said second flow pattern, whilst simultaneously tending to maximise the degree of transversality in the crossing of streamlines, the twist function or velocity profile of a given flow pattern acts in the same direction over a greater area of the working region. This configuration advantageously alleviates any inherent non-monotonicity in the velocity profile of the flow pattern.

Alternatively, the supply means and removal means may be respectively modelled by a "line" source or sink.

For example, a supply means which is representative of a line source, may advantageously comprise a channel or vessel which is in fluid communication (for example by means of a single opening or a plurality of closely spaced openings) with the chamber along the length of the channel, and which is operable to supply fluid having a particular velocity profile to the chamber. Preferably, the velocity profile generated by the supply means increases monotonically across the chamber. The peak in the velocity profile may preferably be substantially aligned along the boundary of the chamber. In this case, the twist function generated by the line

source-sink pair will not exhibit a turning point and thus, the supply means and removal means are ideally configured such that the velocity function of the flow pattern increases/decreases monotonically throughout the whole chamber.

According to a particularly preferred embodiment, there is provided an apparatus provided with first and second supply means, each supply means comprising a a line source, and first and second removal means, each removal means comprising a line sink. Preferably, the apparatus comprises a chamber having a square shaped perimeter, wherein the first and second supply means are provided substantially along first and second adjacent orthogonal edges of the chamber, and wherein each removal means is positioned along the edge opposing the corresponding supply means. Each supply means is operable to supply a substance to the chamber having a velocity profile which varies (increases/decreases) monotonically across the chamber.

Thus, according to this embodiment, the streamline which represents the motion of particles which travel from source to sink in the shortest time (which will be substantially equal in length to the other streamlines of the flow pattern) is located substantially along one edge of the chamber, whilst the streamline which represents the motion of particles which travel from source to sink in the longest time ("longest streamline") is located substantially along the opposite edge of the chamber.

According to a second aspect of the present invention, there is provided a method of in accordance with the features of claim 15.

Thus, according to embodiments of the present invention which provide a method of designing an apparatus for inducing motion of particles of one or more substances, it is necessary to try to balance the desired conditions for a particular set of physical conditions relating to the shape and size of the chamber and any physical characteristics of the mixing apparatus.

As an alternative to step iv) of the mentioned method, the method may include the step of generating a representation of the apparatus design, which may be generated for visualisation on an electronic display device, stored on a medium for subsequent retrieval, embedded within an electrical signal or printed as a hard copy.

The present invention also provides computer programs and computer program products for carrying out any of the methods described herein, and computer readable media having stored thereon programs for carrying out any of the methods described herein. A computer program embodying the invention may be stored on a computer-readable medium, or it could, for example, be in the form of a signal such as a downloadable data signal provided from an Internet web site, or it could be in any other form.

Embodiments of the present invention have been shown to demonstrate a significant reduction in the occurrence of islands of unmixed fluid i.e., regions of "trapped" fluid which do not interact/mix with the surrounding fluid, and is thus more efficient mixing over a greater extent of the chamber. In particular, analysis of a number of apparatuses for inducing motion of particles of one or more substances which have been designed according to embodiments of the present invention, have been shown to exhibit chaotic particle trajectories over the whole working area of the chamber, even when the pumping time is low. Indeed, the present inventors have demonstrated from the analysis conducted for the previously considered mixing protocols, which all give rise to non-monotonic velocity profiles, that a central parabolic island of unmixed fluid should be expected at the point at which the twist function breaks down. According to embodiments of the present invention, this point is moved to the edge of the chamber, thereby ensuring the a greater extent of the chamber, and particularly the working region (typically the central region of the chamber) exhibits chaotic particle trajectories.

Within the context of a DNA micro-array, embodiments of the present invention are advantageous in that the presence of islands of unmixed fluid can be advantageously eliminated within the working region of the chamber so that the need to rely on molecular diffusion in some areas is alleviated. Hybridization therefore occurs more efficiently and quickly and is more reproducible and more accurate.

The supply and removal means may be positioned such that the shortest streamline of the first and second flow pattern runs substantially adjacent transverse boundaries of the chamber. The chamber may be circular, or rectangular or any other shape.

The supply means and/or removal means may comprises one or more syringes which are operable to inject or remove fluid from the chamber. Preferably, the apparatus utilises one or more syringes in conjunction with a means, such as a tube, for connecting the syringes. One or more valves may be provided to allow the supply and removal means to be selectively operated as first or second source-sink pairs.

Preferably, the apparatus of the present invention, or designed according to a method of the present invention, comprises a DNA microarray.

It is envisaged that embodiments of the present invention will find useful application to technological applications which involve mixing of particulate materials such as food stuffs and pharmaceuticals. Such applications rely upon mechanisms for facilitate granular flow. Thus, within the context of the present invention, it should be appreciated that references to "flow" or "flow of particles" encompasses fluid flow and granular flow. For a better understanding of the present invention, and to show how the same may be carried into effect, reference will now be made by way of example to the accompanying drawings in which:

Figure 1 illustrates flow patterns arising from source-sink pairs;

Figure 2 show Poincare sections generated for a first previously considered mixing protocol;

Figure 3 show Poincare sections generated for a second previously considered mixing protocol;

Figure 4 illustrates an apparatus according to a first embodiment of the present invention;

Figure 5 illustrates an apparatus according to a second embodiment of the present invention;

Figure 6 shows Poincare sections generated for the first and second embodiments;

Figure 7 illustrates an apparatus according to a third embodiment of the present invention;

Figure 8 illustrates an apparatus according to a fourth embodiment of the present invention;

Figure 9 demonstrates that the flow of a source-sink pair is topologically equivalent to the form of a map on a cylinder; and

Figure 10 show maps of particle motion for first embodiment generated according to an alternative numerical scheme.

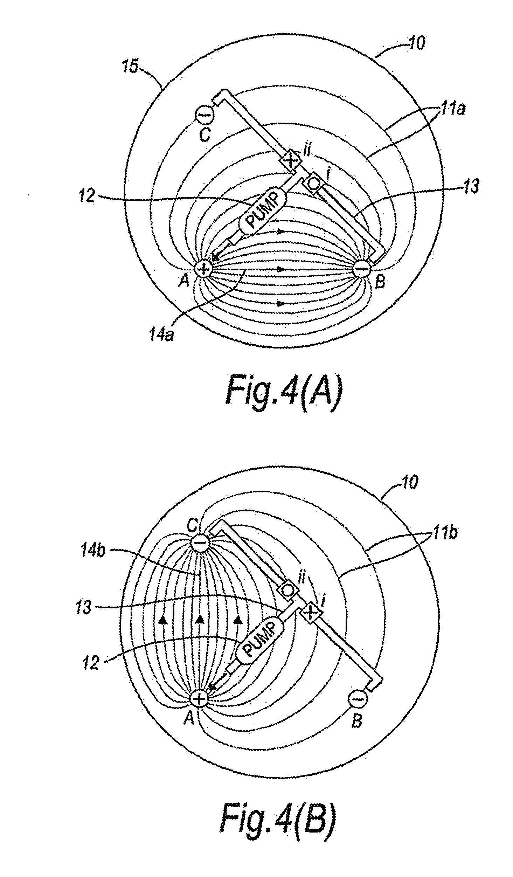

An apparatus 10 according to an embodiment of the present invention, or designed according to an embodiment of the present invention, is shown in Figures 4A and 4B which respectively illustrate first and second flow patterns, 11 a and 11 b, arising from the alternate operation of supply means A with removal means B and supply means A with removal means C. The working region of the chamber is defined as (virtually) the whole chamber. The supply means and removal means therefore act as first and second source-sink pairs and, according to this embodiment, both sources share the same node. It can be seen that the supply means and the removal means are configured such that the source sink pairs are located in an outer region of the chamber i.e. closer to the edge of the chamber than the centre. The position of the source sink pairs may be given by:

$$S_1 = \{S_+(-1,-1),S_-(1,-1)\}$$

$$S_2 = \{S_+(-1,-1),S_-(-1,1)\}$$

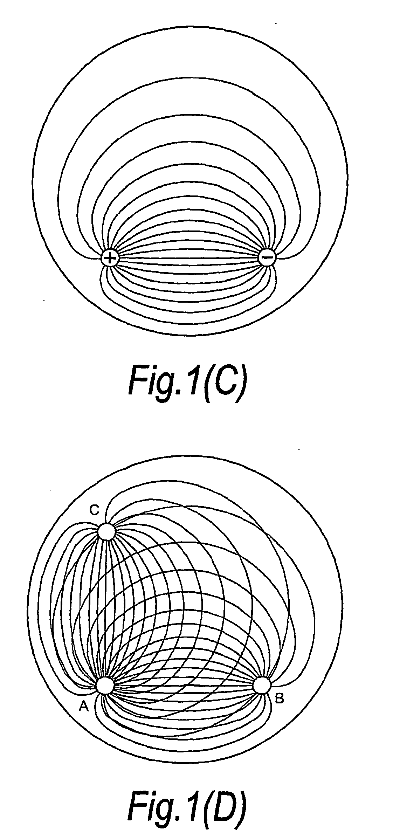

Figure 1D is an illustration of the superimposed flow pattern that arises according to this embodiment.
The apparatus comprises a substantially circular chamber 15, a pump 12 and a tube 13 equipped with valves i and ii. The pump may comprise a peristaltic pump. In use, the pump is used to force fluid into the mixing chamber. The tube 13 connects nodes A, B and C, and the pump 12 injects fluid into A and extracts it via either B or C, depending on the state of the valves. For the first half of the pumping cycle, shown in Figure 4A, valve i is open and valve ii is closed, and node A becomes a source paired with a sink at B. For the second half of the cycle; shown in Figure 4B, valve i is closed and valve ii is open, so that the source at A is paired with a sink at C.
The shortest streamline that connects source A with sink B is 14a and the shortest streamline that connects source A with sink C is 14b. According to this embodiment, the shortest streamlines are separated by an angle of approximately ninety degrees. It can be seen that the direction of the twist function or "shear" of each of the first and second flow patterns remains the same in the central area of the chamber. This has the effect of reducing the non-monotonicity inherent in the flow field arising from the source-sink pairs and breaks the symmetry in the twist function over the chamber.

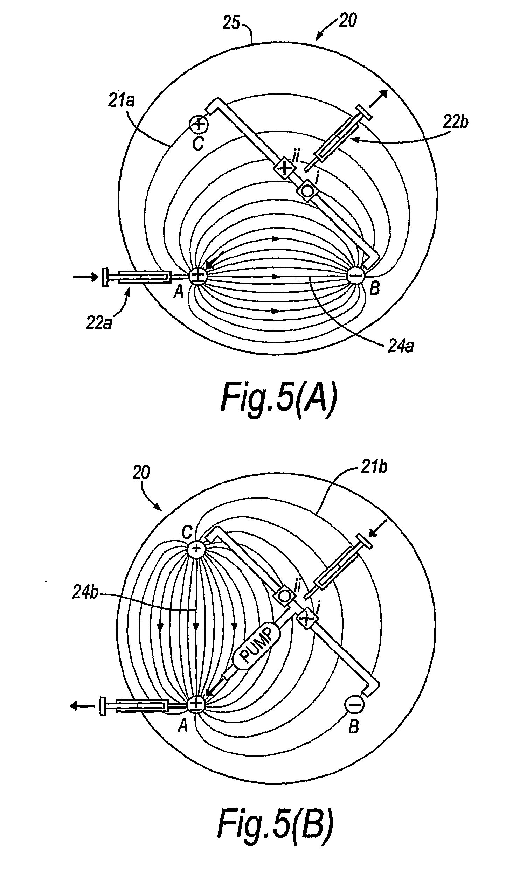

An apparatus 20, according to a second embodiment of the present invention or designed according to an embodiment of the present invention, is shown in Figures 5A and 5B which respectively illustrate first and second flow patterns, 21 a and 21 b, arising from the alternate operation of supply means A with removal means B and supply means C with removal means A. Thus, in this embodiment, the shared node acts first as a source and then as a sink. The position of the source sink pairs may be given by:

$$S_1 = \{S_+(-1,-1), S-(1,-1)\}$$

$$S_2 = \{S_+(-1,+1), S_-(-1,-1)\}$$

Figure 1D is an illustration of the superimposed flow pattern that arises according to this embodiment.

The apparatus comprises a substantially circular chamber 25. Source-sink flows are created via a pair of syringes 22a and 22b. Syringe 22a acts directly into or out of a node marked A, while syringe 22b can act via node 8 or node C depending whether valves (marked i and ii) in a connecting tube are open or closed. For example, during the first half of the pumping cycle, shown in Figure 5A, a source is required at A and a sink at B. Valve i is opened and valve ii is closed, and when fluid is injected via syringe 22a and extracted via syringe 22b, the desired source-sink flow is achieved. After the pumping time T has elapsed, node C is required to become a source and node A to become a sink. Valves i and ii are thus switched, so that valve i is closed and valve ii is open, and the fluid now stored in syringe 22b is injected via the source at C and extracted into syringe 22a at A. This procedure is then repeated cyclically.

The shortest streamline that connects source A with sink 8 is 24a and the shortest streamline that connects source C with sink A is 24b.

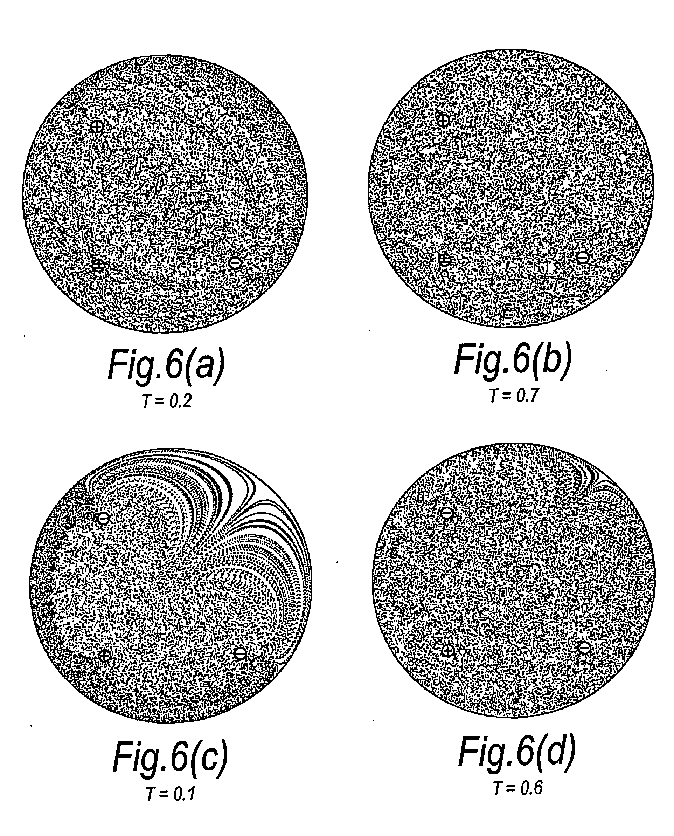

Figure 6 shows Poincare sections generated for the first and second embodiments at pumping time T= 0.1 and T = 0.6 (first embodiment) and pumping time T = 0.2 and T = 0.7 (second embodiment). It can be seen that, even for very short pumping times, no islands of unmixed fluid are seen within the chamber. Thus, both embodiments are demonstrated to show excellent ergodic mixing properties for short and long pumping times.

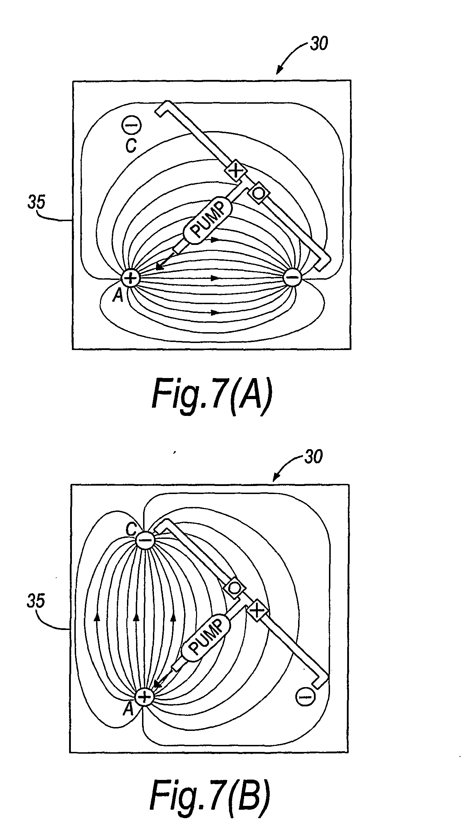

Figure 7 shows an apparatus 30 according to a third embodiment of the present invention. The apparatus is similar in fabrication to the apparatus of the first embodiment, in that both sources share the same node and in that the positions of the source sink pairs may be given by:

$$S_1 = \{S_+(-1,-1), S_-(1,-1)\}$$

$$S_2 = \{S_+(-1,-1), S_-(-1,1)\}$$

Once again, the superimposed flow pattern that arises according to this embodiment is shown in Figure 2d. However, according to the third embodiment, the apparatus comprises a square chamber 35.

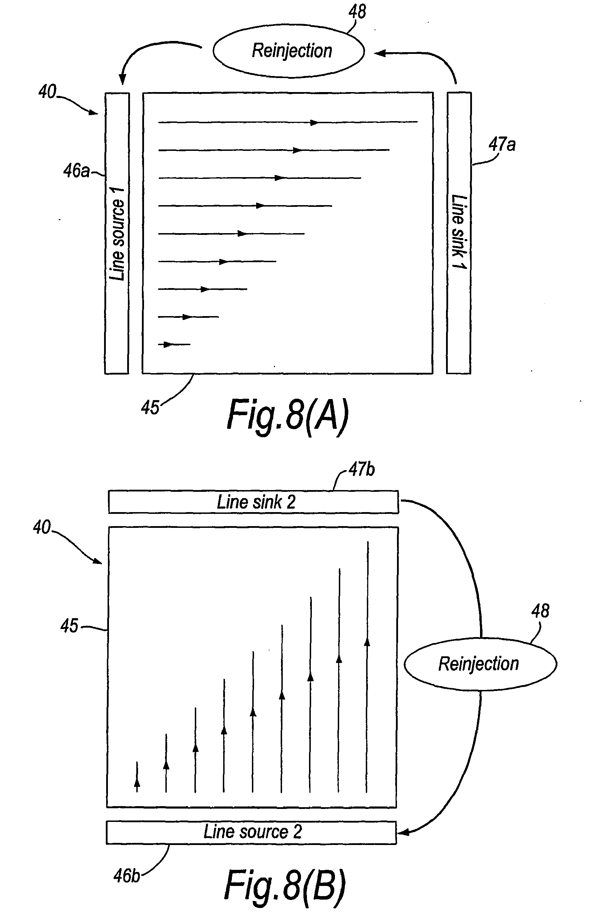

Figure 8 illustrates a design for a hybridization chamber according to the present invention. According to this embodiment, the apparatus 40 comprises a square shaped chamber 45, the perimeter of which defines a working region, and is provided with two line sources 46a and 46b, which comprise the supply means of the apparatus, and two sinks 47a and 47b which comprise the removal means of the apparatus. The line sources are positioned along orthogonal edges of the chamber and are operable to supply a substance to the chamber so as to create a steady shear flow with streamlines illustrated by the arrows. The apparatus is further provided with a reinjecting means 48, which may take the form of a pump and one or more tubes in fluid communication with both the line source and the line sink, such that particles of a substance which have left the chamber via the sink are re-injected back into the chamber. In use as a DNA micro-array, a plurality of DNA strands are attached to a glass or silicon substrate which is placed in the chamber. The chamber contains a solution of mRNA which is cyclically supplied to and removed from the chamber by the supply and removal means so as to keep the volume of mRNA substantially constant.

The fabrication of a supply means which comprises a line source may be achieved in a number of ways as will be appreciated by those skilled in the art. For example, the supply means may comprise a channel or a vessel having plurality of closely spaced openings, each in fluid communication with one edge of the chamber, or it may comprise a single opening which runs substantially along the length of the line source in fluid communication with the chamber. It is envisaged that the apparatus may be provided with a means to create a pressure differential along the length of the line source so that fluid particles are forced into the chamber at different speeds in order to create the required shear flow. This differential may be created by means of a plurality of valves positioned along the length of the source, in order to create a pressure profile which decreases monotonically along the length of the line source. Applying a pressure to only one end of the line source will also result in a gradual pressure drop over the length of the line source.

The line sink may similarly comprise a channel or a vessel having a plurality of closely spaced openings, each in fluid communication with the chamber. It may be provided with a pumping means operable to draw fluid from the chamber in order to create the required velocity profile.

It can be appreciated that an apparatus of said embodiment is operable to create a first and second flow pattern which exhibits a substantially monotonic velocity field.

**Theoretical Analysis**

In this section we shall provide some theoretical justification for the features of the present invention which have been shown to exhibit an improved mixing efficiency.

**1. Flow-patterns arising from source-sink pairs**

The justification for the flow generated by source-sink pairs as a model for viscous flow injected and removed from, for example, a DNA microarray hybridization chamber is described in number publications, for example, an article entitled "Chaotic mixer improves microarray hybridisation" by McQuain, M. K., Seale, K., Peek, J., Fisher, T. S., Levy, S., Stremler, M. A., & Haselton, F. R and published in Analytical Biochemistry, 325, 215-226.

The following theoretical background is useful for understanding the background work that has led to the present invention, in particular for understanding how the flow pattern, or streamline pattern, representing the motion of particles from a source to the corresponding sink can be determined.

It is assumed that a particle which escapes through a sink is re-injected through the corresponding source.

The situation for unbounded fluid flow is relatively simple, and much can be computed analytically. Without loss of generality, place a source $S_+$ of strength $Q$ at (-a, 0) in Cartesian coordinates, and a corresponding sink $S-$ of strength $- Q$ at (a, 0). The velocity potential $\varphi$ and stream function $\psi$ are the real and imaginary parts of the complex potential:

$$W(z) = \phi + i\psi = \frac{Q}{2\pi}\left(\log(z+a) - \log(z-a)\right) \tag{1}$$

Writing $z = x(t) + iy(t)$, the equations of motion are then given by.

$$\left(\dot{x}(t), \dot{y}(t)\right) = \nabla\phi = \left(\delta\psi/\delta y, -\delta\psi/\delta x\right) \tag{2}$$

Streamlines are lines of constant $\psi$, and under the influence of a single source-sink pair, the flow is steady and so fluid particles are constrained to move along a given streamline. These are easily computed to be circular arcs.

Such a source-sink system is specified by two parameters, namely the distance $2a$ between source and sink, and the source/sink strength $Q$. The first of these defines the size of the system, and the second is responsible for the speed of its evolution. Using a time unit of $2\pi a^2/Q$ produces a dimensionless system with a single parameter. The time taken for a particle to travel from source to sink will be an important quantity. This time is referred to as the transfer time, $\tau$ and depends on the particular streamline along which the particle travels. The shortest possible transfer time as the direct transfer time, $\tau_c$, and, for the unbounded case, it is clear that the streamline giving rise to the direct transfer time is the straight line connecting source and sink. For other streamlines, the transfer time is a function of the angle $\alpha$ measured from the straight line connecting source and sink to the tangent at which a particle leaves the source. Using the above non-dimensionalisation, the direct transfer time can be found by integrating equation E along the line $y = 0$ between $x = - a$ and $x = a$, giving $\tau_c = \tau(0) = 2a^2/3$. In particular, when the source and sink are placed at ( -1, 0) and (1,0) respectively, $T_c = 2/3$.

Physical considerations require a boundary. If a circular boundary is assumed, we introduce image sources and sinks according to the Milne-Thomson circle theorem (see, for example, Batchelor, G. K. (1967). An Introduction to Fluid Dynamics. Cambridge University Press.). This states that given a complex potential $W(z) = f(z)$ such that all singularities of $f(z)$ the outside the circle $|z| = R$, then the potential

$$W(z) = f(z) + \overline{f}(R^2 / z)$$

$(3)$

(where the bar indicates that i has been replaced by $-i$ except in $z$ itself) produces a flow with no new singularities outside $|z| = R$, and where the circle $|z| = R$ itself is a streamline. This theorem is usually applied to situations such as computing flow past a cylinder. Introducing a circular boundary of radius $R > a$ centred at the origin to the source-sink pair described above creates a pair of image singularities outside $|z| = R$, and also two image singularities at the origin. However, because the source and sink are of equal strength, the singularities at the origin cancel out, and the theorem produces a valid potential for which the boundary is a streamline. In particular, introducing a circular boundary of radius $R$ to the potential given in (71) results in the potential

$$W(z) = \phi + i\psi = \frac{Q}{2\pi}\left(\log(z + a) - \log(z - a) + \log(R^2 + az) - \log(R^2 - az)\right)$$

$(4)$

Streamlines for this potential are no longer circular arcs, but are shown in Figure 1A for the case a = 1, $R = 2$. For this source-sink system, using the nondimensionalization above, the direct transfer time $T_c$ can be computed in the same manner as for the unbounded case, giving $\tau_{\varepsilon}$, = $r(0)$= 38/15 - 9log(3)/5 $\approx$ 0.556.

The same procedure can be used for sources and sinks which are not placed diametrically. Consider, for example, a source placed at ( -1, -i) and a sink placed at (1, -i) in the complex plane. This has complex potential given by

$$W(z) = \phi + i\psi = \frac{Q}{2\pi}\left(\log(z + 1 + i) - \log(z - 1 + i) + \log(R^2 + (1 - i)z) - \log(R^2 - (1 + i)z)\right)$$

$(5)$

Streamlines for this system, with $R = 2$ are shown in Figure 1(c). Numerically, the direct transfer time can be found to be $T_c$ « 0.51.

In the case of a non-circular boundary the above method together with conformal mappings can be used to produce flow patterns for each source-sink pair.

### 2. First numerical consideration of particle motion

A first numerical scheme for considering the fluid particle motion is as follows. Starting from a given initial condition, the velocity field given by the source-sink pair S1 for a pumping time T is integrated. If the particle enters the sink during this time, it is assumed to be re-injected from the corresponding source. After time T the velocity field resulting from source-sink pair S2 are integrated in the same way, and so on. As previously illustrated, the motion of particles under the velocity fields generated by source-sink pairs can be illustrated by generating discrete time Poincare sections.

### 3. Alternative numerical consideration of particle motion

In addition to generating Poincare sections for the previously considered protocols/devices, the present inventors have also sought to provide concrete mathematical and numerical conclusions about the efficiency of mixing in these prior protocols using a kinematical model based on a mathematical framework known as the linked twist map. This mathematical framework develops the analogy of a source-sink pair as a model for the flow field generated by the cyclic removal and reinjection of particles to a chamber.

Considering a flow that takes place in a square with doubly periodic boundary conditions. Physically, this means that fluid that leaves the square through x = 1 is reinjected at the same y value at x = 0. Similarly, fluid that leaves the square at y = 1 is re-injected at the same x value at y = 1. Mathematically, the flow is said to take place on a two-dimensional torus. More precisely, consider the unit square, 0<x<1,0<y<1, where we "identify" x = 0 with x = 1 and y = 0 with y = 1.

The resultant flow is a unidirectional flow on this square with velocity field given by:

$$\frac{dx}{dt} = v(y), \quad \frac{dy}{dt} = 0 \tag{6}$$

The function v(y) is referred to as the velocity profile, and it is unspecified so far. If v(y) is a monotone function then (6) is a parallel shear flow. The velocity field (6) is easily integrated to solve for the fluid particle motions. The trajectory of a particle starting at $(x_0, y_0)$ at t = 0 is given by:

$$x(t) = x_0 + v(y_0)t, \quad y(t) = y_0. \tag{7}$$

It follows that after a fixed time T the particle has moved from $(x_0, y_0)$ to $(x(T), y(T))$ $(x_1, y_1) = (x_0 + v(y_0)T, y_0)$. More generally, if $(x_n, y_n)$ denotes the location of the particle after time nT (where n is a positive integer), then the map describing the motion of particles is given by:

$$x_{n+1} = x_n + Tv(y_n), \quad y_{n+1} = y_n. \tag{8}$$

Mathematicians refer to this as a twist map. Note that under this flow material simply tends to become aligned with the x-axis, and so does not mix exponentially quickly. Mixing can be created by periodically re-aligning the flow in a direction perpendicular to the streamlines of equation (6). Mathematically, this is achieved as follows. After some fixed time T, modify the flow so that it has the form:

$$\frac{dx}{dt} = 0, \quad \frac{dy}{dt} = v(x). \tag{9}$$

Now by the same reasoning as above, the motion of fluid particles can be described by the following map:

$$x_{n+1} = x_n, \quad y_{n+1} = Tv(x_n) + y_n \tag{10}$$

We adopt the shorthand notation $x \equiv (x,y)$, and denote (8) by $x_{n+1} = Hx_n$ and (10) by $x_{n+1} = Vx_n$. Then the motion of fluid particles under the flow obtained by periodically switching between (6) and (9) is described repeated application, or "iteration" of the following map:

$$x_{n+1} = VHx_n \tag{11}$$

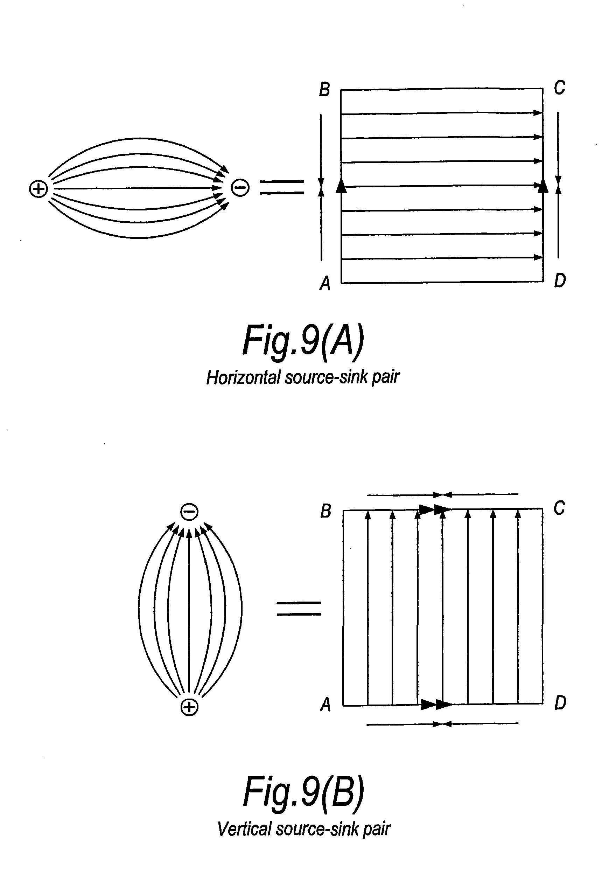

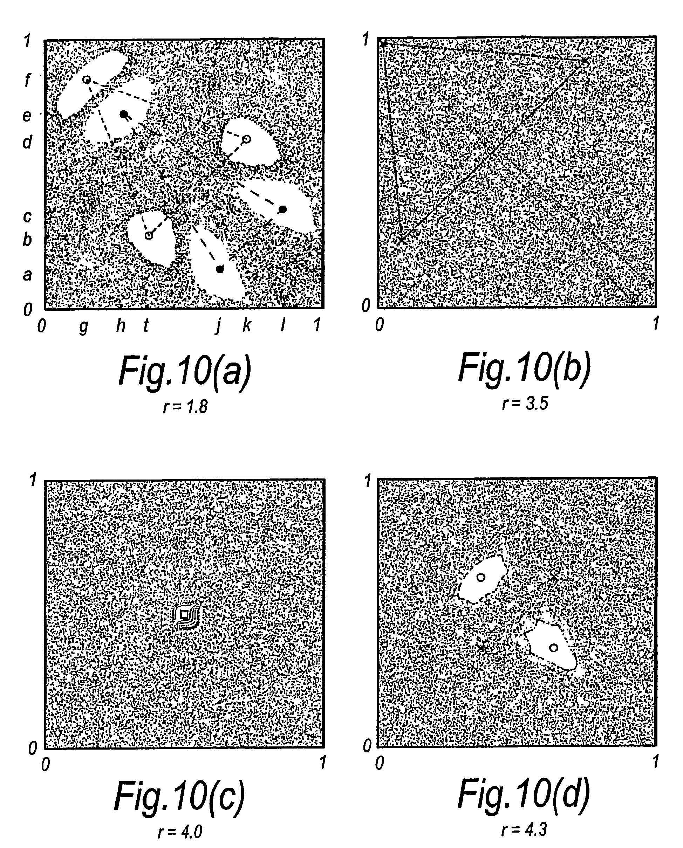

The flow represented by equation (11) has been referred to as eggbeater flow since it represents a simplified model of a hand held eggbeater. Mathematically, this is known as a linked twist map. The linked twist map embodies the paradigm of "streamline crossing" which, in some senses, is the primary mechanism for creating chaotic advection.
Referring to Figure 9, it can be appreciated that the flow of a source-sink pair is topologically equivalent to the form of a map on a cylinder by recognizing that the mechanism of making fluid which disappears down a sink appear at a source is equivalent to the identification of opposite sides of a square that is made in the construction of a cylinder (i.e., "periodic boundary conditions"). If one re-injects fluid from a source at the same angle as it entered the sink then the analogy is simple - a source-sink pair is topologically equivalent to a twist map on a cylinder. More precisely, referring to Figure 9, the unit square is defined as *U* = [0,1] x [0,1] in Cartesian coordinates (*x,y*), with corners *A, B, C, D. A* cylinder *Ch* can be constructed by identifying the edge *AB* with the edge *DC*. Horizontal lines (lines of constant *y*) become circles under this identification. By 'pinching' the edges *AB* and *DC* down to a single point as in Figure 10 (that is, identifying all points on *AB* and *DC*), the horizontal circles on the cylinder represent streamlines in the source-sink model.

To ensure that trajectories of this map are constrained to a given streamline, the map on the cylinder *Ch* is given by *F(x,y) = (x + f(y),y)*. Similarly, a source-sink pair placed at right angles to the original pair can be naturally described as a map *G(x,y) = (x,* y+ g(x)) on a cylinder $C_v$, as shown in Figure 1(b). A particle entering a sink on a given streamline is assumed to reappear from a source on the same streamline.

This alternative numerical scheme corresponds to alternating the action of map *F* on the cylinder *Ch* with that of the map *G* on the cylinder *Cy*. The resulting dynamical system is a map *H* on the torus $T^2$ given by the composition of *F* with *G*.

This alternative numerical scheme allows the performance of an apparatus, such as a DNA microarray, to be considered and also allows some concrete mathematical and numerical conclusions to be reached which help explain the significance and importance of the features of the present invention.

Firstly, the linked twist map$H : T^2 {\rightarrow} T^2$ is by:

$$H(x,y) = G \circ F(x,y) \qquad (12)$$

where the maps $F : T^2 {\rightarrow} T^2$ and $G : T^2 \rightarrow T^2$ have the forms

$$F(x, y) = (x + f(y), y). \qquad (13)$$

$$G(x, y) = (x, y + g(x)) \qquad (14)$$

as described above. The form of the twist functions depends on the geometry of the source-sink protocol in question, and some issues regarding this are discussed in the following section. The performance of the a given mixing protocol, or apparatus design, can be assessed by using this alternative model to create stroboscopic sections in order to map the dynamics of particles in the system. In this case, iterates of H correspond to iterates of a Poincare map. Thus, considering again the previously considered mixing protocol wherein two source-sink pairs are placed symmetrically in a circular boundary of radius 2 centred at the origin, with positions given by:

$$S_1 \quad = \quad \{S_+(-1,0), S_-(1,0)\}$$

$$S_2 \quad = \quad \{S_+(0,-1), S_-(0,1)\}$$

Thus, F(x, y) = (x + f(y), y) and G(x, y) = (x, y + g(x)), with f(y) = ry(1 - y) and g(x) = rx(1 - x). The behaviour of this protocol is considered by plotting 20000 iterates from a single initial condition. The resultant maps are shown in Figure 10.

On increasing the parameter r the map displays qualitatively the same behaviour as the Poincare sections. In particular, comparing with Figure 2, we see that for small values of r (for example, r =1.8 in Figure 2a) islands occur over a significant proportion of the domain. When the parameter is increased to r = 3.5, as in Figure 10(b) (c.f. Figure 2 (b)), the system appears well mixed. The parameter r = 4.0 is a critical parameter value (see Figure 2(c)). At this value, the central point *(x,y)* = (1/2,1/2) is a fixed point for both F(x,y) and G(x,y). This corresponds to the value of T in the original model which returns a particle in the centre of the domain to this position after exactly one pumping cycle. As in the original model, at this critical parameter a central island begins to appear. On increasing r further, the central island enlarges, and then splits in two, in this case (see Figure 2(d)) forming a pair of islands located on the off-diagonal of the torus. As for the Poincare sections, the central island occurs for integer multiples of the critical parameter, that is, r = 4,8,12,..., and each time splits into a pair of off-diagonal islands upon increasing r.

However, if f and g are chosen to be monotonic increasing functions such that f(0) = g(0) = 0, and f(1) = k, and g(1) = I, where k and I are integers (in order to preserve continuity at the identified edges of the torus) of the same sign (the co-rotating case), H can be shown to be ergodic, mixing, and possess the Bernoulli property. For any pair of open sets $A, B \subset T^2$

$$\lim_{n\to\infty} \frac{\mu\left(H^n(A)\right)\cap B}{\mu(B)} = \mu(A), \qquad (15)$$

where $\mu(\cdot)$ represents Lebesgue measure (i.e., roughly, the area of a set) and Hn denotes the nth iterate of H. The meaning of this definition is as follows. Suppose the set A is occupied by the target and it occupies 20% of the hybridization chamber (note that the area of the hybridization chamber is normalized to unity, i.e. $\mu(T^2)$ = 1). After n cycles of operation of the source-sink pairs the fraction of target in the part of the hybridization chamber denoted by the set B is $\mu(H^n(A))$ $\cap B/\mu(B)$. Under repeated cycling of the source-sink pairs (i.e., in the limit as n→ ∞) the fraction of target in B approaches 20%, i.e., $\mu$ (A). Since B is chosen arbitrarily, this means that the target will have spread uniformly throughout the hybridization chamber.

The fact and proof of mixing for such a system depends crucially on the monotonicity of the twist functions, and the fact that the twists act in transverse directions everywhere. These are the critical features which preferred embodiments of the present invention seek to achieve.

On a theoretical level, the present inventors have therefore shown that a LTM which represents two superimposed flow field patterns is shown to be ergodic, mixing and possess the Bernoulli property if the twist functions are both monotonic and relatively transverse. This explains why embodiments of the present invention which seek to minimise any non-monotonicity in the twist function and to maximise the degree of transversality in the crossing of streamlines (and thus the twist functions) have been shown to demonstrate chaotic particle trajectories everywhere within the working region of a chamber, even when pumping time T is relatively low.

**Claims**

1.  An apparatus (10, 20, 40) for inducing motion of particles of one or more substances, the apparatus comprising a chamber (15, 25, 45) having a supply means, operable to supply a substance to the chamber (15, 25, 45), and a removal means, operable to remove a substance from the chamber (15, 25, 45), said chamber (15, 25, 45) having a working region, wherein said supply means and said removal means are further operable to represent first and second source-sink pairs, each source-sink pair comprising a source and a corresponding sink, and wherein said supply means and said removal means are configured:

    such that streamlines of a first flow pattern (11 a, 21 a), which streamlines represent the motion of particles travelling from the source of said first source-sink pair to the corresponding sink of said first source-sink pair, cross streamlines of a second flow pattern (11 b, 21 b), which streamlines represent the motion of particles travelling from the source of said second source-sink pair to the corresponding sink of said second source-sink pair;
    **characterised in that** the streamline (14a, 24a) of the first flow pattern (11 a, 21 a) which represents the particles travelling with the greatest velocity, defines a working angle of between 60 and 120 degrees with the streamline (14b, 24b) of the second flow pattern (11 b, 21 b) which represents the particles travelling with the greatest velocity, and **in that** the streamline (14a, 24a) of the first flow pattern (11 a, 21 a) which represents the particles travelling with the greatest velocity, does not cross the streamline (14b, 24b) of the second flow pattern (11 b, 21 b) which represents the particles travelling with the greatest velocity in a central region of the working region, such that:

    a) the degree of transversality in the crossing of streamlines of said first (11a, 21 a) and second (11 b, 21 b) flow patterns within said working region; and b) the area over which the velocity function of said first (11a, 21 a) and second (11b, 21b) flow patterns varies monotonically within said working region, are mutually maximised.

2.  An apparatus (10, 20) as claimed in claim 1, further comprising control means operable such that said supply means and said removal means are selectively operable to alternately represent said first and second source-sink pairs.

3.  An apparatus (10, 20) as claimed in claim 1 or 2, wherein said first and second source-sink pairs each comprise a point source and a point sink.

4.  An apparatus (20) as claimed in claim 3, wherein said supply means comprises one or more syringes (22a, 22b), operable to inject a substance into the chamber (25), and wherein said removal means comprises one or more syringes (22a, 22b) operable to remove a substance from the chamber (25).

5. An apparatus (40) as claimed in claim 1 or 2, comprising first and second supply means and first and second removal means, wherein each supply means comprises a line source (46a, 46b) and each removal means comprises a line sink (47a, 47b).

6. An apparatus (40) as claimed in claim 5, wherein said supply means comprises means to supply a fluid to the chamber (45) such that the velocity profile of the fluid varies monotonically across the working region of the chamber (45).

7. An apparatus (10, 20) as claimed in any preceding claim, wherein the chamber (15, 25) has a perimeter which is substantially circular in shape.

8. An apparatus (40) as claimed in any one of claims 1 to 6, wherein the chamber (45) has a perimeter which is substantially square or rectangular in shape.

9. An apparatus (40) as claimed in claim 8, when appended to claim 5 or 6, wherein said first and second supply means are provided substantially along first and second adjacent orthogonal edges of the chamber (45), and wherein each removal means is positioned along the edge opposing the corresponding supply means.

10. An apparatus (10, 20, 40) as claimed in any preceding claim, wherein the apparatus comprises a DNA microarray.

11. An apparatus (10, 20) as claimed in claim 1, wherein said supply means and said removal means consists of three nodes which are operable to comprise said first and second source-sink pairs, wherein one said node is a common node, which common node comprises a source or a sink of said first source-sink pair and a source or a sink of said second source-sink pair,
wherein said nodes are positioned such that first (24a) and second (24b) shortest lines, which shortest lines (24a, 24b) represent the shortest distance between the common node and each of the other nodes, mutually define said working angle of between 60 and 120 degrees, wherein said nodes are positioned with respect to the chamber (15, 25) such that said working angle acts over the majority of the chamber (15, 25) including the central region thereof.

12. An apparatus (10, 20) as claimed in claim 1, wherein said supply means and said removal means consists of three nodes which are operable to comprise said first and second source-sink pairs, wherein one said node is a common node, which common node comprises a source or a sink of said first source-sink pair and a source or a sink of said second source-sink pair, wherein said nodes are positioned such that first (24a) and second (24b) shortest lines, which shortest lines (24a, 24b) represent the shortest distance between the common node and each of the other nodes, mutually define said working angle of between 60 and 120 degrees, wherein all said nodes are positioned at, or near to, the outer region of the chamber (15, 25).

13. An apparatus (40) as claimed in claim 1, wherein said supply means and said removal means are positioned such that a first shortest line between the source of the first source-sink pair and the corresponding sink is substantially orthogonal to a second shortest line between the source of the second source-sink pair and the corresponding sink, said first and second shortest lines representing the shortest distance between one said source and the corresponding sink, wherein said chamber (45) is square or rectangular in shape and wherein said first and second shortest lines extend substantially alongside adjacent orthogonal edges of the chamber.

14. An apparatus (10, 20) as claimed in claim 1, wherein said supply means and said removal means are configured such that a first shortest line (24a) between the source of the first source-sink pair and the corresponding sink defines said working angle of between 60 and 120 degrees with respect to a shortest line (24b) between the source of the second source-sink pair and the corresponding sink, said first and second shortest lines (24a, 24b) representing the shortest distance between the respect source and the corresponding sink, wherein said chamber (15, 25) is circular in shape and wherein said supply means and said removal means are configured such that the point of intersection, or the projected point of intersection, of said shortest lines (24a, 24b) is at or near the outer region of the chamber (15, 25), and such that said working angle acts over the majority of the chamber (15, 25) including the central region thereof.

15. A method of designing an apparatus (10, 20, 40) for inducing motion of particles of one or more substances, the apparatus (10, 20, 40) comprising a chamber (15, 25, 45) having a supply means, operable to supply a substance to the chamber (15, 25, 45), and a removal means, operable to remove a substance from the chamber (15, 25, 45), wherein said chamber (15, 25, 45) comprises a working region, said supply means and said removal means being further operable to represent first and second source-sink pairs, each source-sink pair comprising a source and a corresponding sink, wherein the method comprises the steps of:

i) determining a first flow pattern (11a, 21a) which represents the motion of particles travelling from the source of said first source-sink pair to the corresponding sink of said first source-sink pair, said first flow pattern (11a, 21 a) comprising a plurality of streamlines;

ii) determining a second flow pattern (11 b, 21 b) which represents the motion of particles travelling from the source of said second source-sink pair to the corresponding sink of said second source-sink pair, said second flow pattern (11 b, 21 b) comprising a plurality of streamlines;

iii) determining the locations of said first and second source-sink pairs within said chamber (15, 25, 45);

**characterised in that** the streamline (14a, 24a) of the first flow pattern (11a, 21 a) which represents the particles travelling with the greatest velocity, defines a working angle of between 60 and 120 degrees with the streamline (14b, 24b) of the second flow pattern (11 b, 21 b) which represents the particles travelling with the greatest velocity, and **in that** the streamline (14a, 24a) of the first flow pattern (11 a, 21 a) which represents the particles travelling with the greatest velocity, does not cross the streamline (14b, 24b) of the second flow pattern (11 b, 21 b) which represents the particles travelling with the greatest velocity in a central region of the working region, such that:

a) the degree of transversality in the crossing of streamlines of said first (11a, 21 a) and second (11 b, 21 b) flow patterns within said working region; and b) the area over which the velocity function of said first (11a, 21a) and second (11b, 21b) flow patterns varies monotonically within said working region, are mutually maximised;

iv) making said apparatus (10, 20, 40) such that said supply means and said removal means are positioned within said chamber (15, 25, 45) so as to represent the locations of said first and second source-sink pairs selected according to step iii).

16. A computer program which, when loaded into a computer, causes the computer to carry out the method of claim 15.

17. A computer program as clamed in claim 16, carried by a carrier medium.

18. A computer program as claimed in claim 16, wherein said carrier medium is a recording medium.

19. A computer program as claimed in claim 16, wherein said carrier medium is a transmission medium.

**Patentansprüche**

1. Vorrichtung (10, 20, 40) zum Hervorrufen der Bewegung von Teilchen einer oder mehrerer Substanzen, wobei die Vorrichtung eine Kammer (15, 25, 45) umfasst, die eine Zuführeinrichtung aufweist, die zum Zuführen einer Substanz in die Kammer (15, 25, 45) betreibbar ist, und eine Abführeinrichtung, die zum Entfernen einer Substanz aus der Kammer (15, 25, 45) betreibbar ist, und die Kammer (15, 25, 45) einen Arbeitsbereich besitzt, und die Zuführeinrichtung und die Abführeinrichtung zudem so betreibbar sind, dass sie erste und zweite Quellen-Sinken-Paare darstellen, wobei jedes Quellen-Sinken-Paar eine Quelle und eine zugehörige Sinke umfasst, und wobei die Zuführeinrichtung und die Abführeinrichtung so konfiguriert sind, dass:

Stromlinien eines ersten Strömungsmusters (11a, 21a), wobei die Stromlinien die Bewegung der Teilchen darstellen, die von der Quelle des ersten Quellen-Sinken-Paars zur zugeordneten Sinke des ersten Quellen-Sinken-Paars fließen, Stromlinien eines zweiten Strömungsmusters (11b, 21b) kreuzen, wobei die Stromlinien die Bewegung der Teilchen darstellen, die von der Quelle des zweiten Quellen-Sinken-Paars zur zugeordneten Sinke des zweiten Quellen-Sinken-Paars fließen;

**dadurch gekennzeichnet, dass** die Stromlinie (14a, 24a) des ersten Strömungsmusters (11a, 21a), die die Teilchen repräsentiert, die mit der höchsten Geschwindigkeit fließen, einen Arbeitswinkel zwischen 60 und 120 Grad gegen die Stromlinie (14b, 24b) des zweiten Strömungsmusters (11 b, 21 b) bestimmt, die die Teilchen repräsentiert, die mit der höchsten Geschwindigkeit fließen, und **dadurch**, dass die Stromlinie (14a, 24a) des ersten Strömungsmusters (11a, 21a), die die Teilchen repräsentiert, die mit der höchsten Geschwindigkeit fließen, die Stromlinie (14b, 24b) des zweiten Strömungsmusters (11b, 21 b) nicht kreuzt, die die Teilchen repräsentiert, die mit der höchsten Geschwindigkeit fließen, und zwar in einem Mittenabschnitt des Arbeitsbereichs, so dass:

a) der Querverlaufsgrad beim Kreuzen der Stromlinien des ersten Strömungsmusters (11a, 21a) und des zweiten Strömungsmusters (11b, 21b) innerhalb des Arbeitsbereichs; und
b) das Gebiet, über dem die Geschwindigkeitsfunktion des ersten Strömungsmusters (11a, 21a) und des zweiten Strömungsmusters (11b, 21b) innerhalb des Arbeitsbereichs monoton variiert,

wechselseitig maximiert werden.

2. Vorrichtung (10, 20) nach Anspruch 1, zudem umfassend eine Kontrollvorrichtung, die so betreibbar ist, dass die Zuführeinrichtung und die Abführeinrichtung gezielt so wirken können, dass sie abwechselnd das erste und das zweite Quellen-Sinken-Paar darstellen.

3. Vorrichtung (10, 20) nach Anspruch 1 oder 2, wobei das erste und das zweite Quellen-Sinken-Paar jeweils eine Punktquelle und eine Punktsinke umfassen.

4. Vorrichtung (20) nach Anspruch 3, wobei die Zuführeinrichtung eine oder mehrere Spritzen (22a, 22b) umfasst, die zum Einspritzen einer Substanz in die Kammer (25) betreibbar sind, und wobei die Abführeinrichtung eine oder mehrere Spritzen (22a, 22b) umfasst, die zum Entfernen einer Substanz aus der Kammer (25) betreibbar sind.

5. Vorrichtung (40) nach Anspruch 1 oder 2, umfassend erste und zweite Zuführeinrichtungen und erste und zweite Abführeinrichtungen, wobei jede Zuführeinrichtung eine Linienquelle (46a, 46b) enthält und jede Abführeinrichtung eine Liniensinke (47a, 47b).

6. Vorrichtung (40) nach Anspruch 5, wobei die Zuführeinrichtung ein Mittel umfasst, mit dem der Kammer (45) ein Fluid so zugeführt wird, dass das Geschwindigkeitsprofil des Fluids über den Arbeitsbereich der Kammer (45) monoton variiert.

7. Vorrichtung (10, 20) nach irgendeinem vorhergehenden Anspruch, wobei die Kammer (15, 25) einen im Wesentlichen kreisförmigen Rand aufweist.

8. Vorrichtung (40) nach irgendeinem der Ansprüche 1 bis 6, wobei die Kammer (45) einen Rand aufweist, der im Wesentlichen quadratisch oder rechteckig ist.

9. Vorrichtung (40) nach Anspruch 8 wenn abhängig von Anspruch 5 oder 6, wobei die erste und die zweite Zuführeinrichtung im Wesentlichen entlang erster und zweiter benachbarter orthogonaler Kanten der Kammer (45) bereitgestellt sind, und jede Abführeinrichtung entlang der Kante angeordnet ist, die der zugeordneten Zuführeinrichtung gegenüberliegt.

10. Vorrichtung (10, 20, 40) nach irgendeinem vorhergehenden Anspruch, wobei die Vorrichtung ein DNA-Mikroarray umfasst.

11. Vorrichtung (10, 20) nach Anspruch 1, wobei die Zuführeinrichtung und die Abführeinrichtung aus drei Knoten besteht, die dazu betreibbar sind, dass sie das erste und das zweite Quellen-Sinken-Paar umfassen, und einer der Knoten ein gemeinsamer Knoten ist, und der gemeinsame Knoten eine Quelle oder eine Sinke des ersten Quellen-Sinken-Paars enthält sowie eine Quelle oder eine Sinke des zweiten Quellen-Sinken-Paars,
wobei die Knoten so angeordnet sind, dass eine erste kürzeste Linie (24a) und eine zweite kürzeste Linie (24b), wobei die kürzesten Linien (24a, 24b) die kürzeste Entfernung zwischen dem gemeinsamen Knoten und jedem der anderen Knoten darstellen, gegenseitig den Arbeitswinkel von 60 bis 120 Grad definieren, und die Knoten bezüglich der Kammer (15, 25) so angeordnet sind, dass der Arbeitswinkel über den größten Teil der Kammer (15, 25) wirkt, und zwar einschließlich des Mittenbereichs der Kammer.

12. Vorrichtung (10, 20) nach Anspruch 1, wobei die Zuführeinrichtung und die Abführeinrichtung aus drei Knoten besteht, die dazu betreibbar sind, dass sie das erste und das zweite Quellen-Sinken-Paar umfassen, und einer der Knoten ein gemeinsamer Knoten ist, und der gemeinsame Knoten eine Quelle oder eine Sinke des ersten Quellen-Sinken-Paars enthält sowie eine Quelle oder eine Sinke des zweiten Quellen-Sinken-Paars, und die Knoten so angeordnet sind, dass eine erste kürzeste Linie (24a) und eine zweite kürzeste Linie (24b), wobei die kürzesten Linien (24a, 24b) die kürzeste Entfernung zwischen dem gemeinsamen Knoten und jedem der anderen Knoten darstellen, gegenseitig den Arbeitswinkel von 60 bis 120 Grad definieren, und alle Knoten am oder nahe am Außenbereich der Kammer (15, 25) angeordnet sind.

13. Vorrichtung (40) nach Anspruch 1, wobei die Zuführeinrichtung und die Abführeinrichtung so angeordnet sind, dass eine erste kürzeste Linie zwischen der Quelle des ersten Quellen-Sinken-Paars und der zugehörigen Sinke im Wesentlichen orthogonal zu einer zweiten kürzesten Linie zwischen der Quelle des zweiten Quellen-Sinken-Paars und der zugehörigen Sinke verläuft, und die erste und die zweite kürzeste Linie die kürzeste Entfernung zwischen einer der Quellen und der zugehörigen Sinke repräsentieren, und die Kammer (45) quadratisch oder rechteckförmig ist, und sich die erste und die zweite kürzeste Linie im Wesentlichen entlang benachbarter orthogonaler Kanten der Kammer erstrekken.

14. Vorrichtung (10, 20) nach Anspruch 1, wobei die Zuführeinrichtung und die Abführeinrichtung so konfiguriert sind, dass eine erste kürzeste Linie (24a) zwischen der Quelle des ersten Quellen-Sinken-Paars und der zugehörigen Sinke den Arbeitswinkel zwischen 60 und 120 Grad bezüglich einer kürzesten Linie (24b) zwischen der Quelle des zweiten Quellen-Sinken-Paars und der zugehörigen Sinke definiert, und die erste und die zweite kürzeste Linie (24a, 24b) die kürzeste Entfernung zwischen der jeweiligen Quelle und der zugehörigen Sinke repräsentieren, und die Kammer (15, 25) kreisförmig ist und die Zuführeinrichtung und die Abführeinrichtung so konfiguriert sind, dass sich der Schnittpunkt bzw. der projizierte Schnittpunkt der kürzesten Linien (24a, 24b) am oder nahe am Außenbereich der Kammer (15, 25) befindet, und so, dass der Arbeitswinkel über den größten Teil der Kammer (15, 25) wirkt, und zwar einschließlich des Mittenbereichs der Kammer.

15. Verfahren zum Entwerfen einer Vorrichtung (10, 20, 40) zum Hervorrufen der Bewegung von Teilchen einer oder mehrerer Substanzen, wobei die Vorrichtung (10, 20, 40) eine Kammer (15, 25, 45) umfasst, die eine Zuführeinrichtung aufweist, die zum Zuführen einer Substanz in die Kammer (15, 25, 45) betreibbar ist, und eine Abführeinrichtung, die zum Entfernen einer Substanz aus der Kammer (15, 25, 45) betreibbar ist, und die Kammer (15, 25, 45) einen Arbeitsbereich besitzt und die Zuführeinrichtung und die Abführeinrichtung zudem so betreibbar sind, dass sie erste und zweite Quellen-Sinken-Paare darstellen, und jedes Quellen-Sinken-Paar eine Quelle und eine zugehörige Sinke umfasst, und das Verfahren die Schritte umfasst:

    i) das Festlegen eines ersten Strömungsmusters (11a, 21a), das die Bewegung der Teilchen darstellt, die von der Quelle des ersten Quellen-Sinken-Paars zur zugeordneten Sinke des ersten Quellen-Sinken-Paars fließen, wobei das Strömungsmuster (11 a, 21 a) eine Anzahl Stromlinien umfasst;

    ii) das Festlegen eines zweiten Strömungsmusters (11 b, 21 b) das die Bewegung der Teilchen darstellt, die von der Quelle des zweiten Quellen-Sinken-Paars zur zugeordneten Sinke des zweiten Quellen-Sinken-Paars fließen, wobei das Strömungsmuster (11 b, 21 b) eine Anzahl Stromlinien umfasst;

    iii) das Festlegen der Orte des ersten und des zweiten Quellen-Sinken-Paars innerhalb der Kammer (15, 25, 45); **dadurch gekennzeichnet, dass** die Stromlinie (14a, 24a) des ersten Strömungsmusters (11a, 21a), die die Teilchen repräsentiert, die mit der höchsten Geschwindigkeit fließen, einen Arbeitswinkel zwischen 60 und 120 Grad gegen die Stromlinie (14b, 24b) des zweiten Strömungsmusters (11b, 21 b) bestimmt, die die Teilchen repräsentiert, die mit der höchsten Geschwindigkeit fließen, und **dadurch**, dass die Stromlinie (14a, 24a) des ersten Strömungsmusters (11a, 21a), die die Teilchen repräsentiert, die mit der höchsten Geschwindigkeit fließen, die Stromlinie (14b, 24b) des zweiten Strömungsmusters (11b, 21 b) nicht kreuzt, die die Teilchen repräsentiert, die mit der höchsten Geschwindigkeit fließen, und zwar in einem Mittenabschnitt des Arbeitsbereichs, so dass:

        a) der Querverlaufsgrad beim Kreuzen der Stromlinien des ersten Strömungsmusters (11a, 21a) und des zweiten Strömungsmusters (11b, 21b) innerhalb des Arbeitsbereichs; und

        b) das Gebiet, über dem die Geschwindigkeitsfunktion des ersten Strömungsmusters (11a, 21a) und des zweiten Strömungsmusters (11b, 21b) innerhalb des Arbeitsbereichs monoton variiert,

    wechselseitig maximiert werden;

    iv) das Gestalten der Vorrichtung (10, 20, 40) derart, dass die Zuführeinrichtung und die Abführeinrichtung innerhalb der Kammer (15, 25, 45) so angeordnet werden, dass sie die Orte des ersten und des zweiten Quellen-Sinken-Paars darstellen, die gemäß Schritt iii) gewählt sind.

16. Computerprogramm, das, wenn es in einen Computer geladen wird, den Computer veranlasst, das Verfahren nach Anspruch 15 auszuführen.

17. Computerprogramm nach Anspruch 16, das von einem Trägermedium getragen wird.

18. Computerprogramm nach Anspruch 16, wobei das Trägermedium ein Aufzeichnungsmedium ist.

19. Computerprogramm nach Anspruch 16, wobei das Trägermedium ein Übertragungsmedium ist.

**Revendications**

1. Appareil (10, 20, 40) pour induire un mouvement de particules d'une ou de plusieurs substances, l'appareil comprenant une chambre (15, 25, 45) comportant un moyen d'alimentation, pouvant être utilisé pour délivrer une substance à la chambre (15, 25, 45), et un moyen de retrait, pouvant être utilisé pour retirer une substance de la chambre (15, 25, 45), ladite chambre (15, 25, 45) comportant une région de travail, dans lequel ledit moyen d'alimentation et ledit moyen de retrait peuvent en outre être utilisés pour représenter des première et deuxième paires de source-collecteur, chaque paire de source-collecteur comprenant une source et un collecteur correspondant, et dans lequel ledit moyen d'alimentation et ledit moyen de retrait sont configurés :

de sorte que les lignes de courant d'un premier motif d'écoulement (11a, 21a), lesquelles lignes de courant représentent le mouvement de particules se déplaçant de la source de ladite première paire de source-collecteur vers le collecteur correspondant de ladite première paire de source-collecteur, croisent les lignes de courant d'un deuxième motif d'écoulement (11b, 21b), lesquelles lignes de courant représentent le mouvement de particules se déplaçant de la source de ladite deuxième paire de source-collecteur vers le collecteur correspondant de ladite deuxième paire de source-collecteur ;
**caractérisé en ce que** la ligne de courant (14a, 24a) du premier motif d'écoulement (11a, 21a) qui représente les particules se déplaçant avec la vitesse la plus grande définit un angle de travail entre 60 et 120 degrés avec la ligne de courant (14b, 24b) du deuxième motif d'écoulement (11b, 21b) qui représente les particules se déplaçant avec la vitesse la plus grande, et **en ce que** la ligne de courant (14a, 24a) du premier motif d'écoulement (11a, 21a) qui représente les particules se déplaçant avec la vitesse la plus grande ne croise pas la ligne de courant (14b, 24b) du deuxième motif d'écoulement (11b, 21b) qui représente les particules se déplaçant avec la vitesse la plus grande dans une région centrale de la région de travail, de sorte que :

a) le degré de transversalité du croisement des lignes de courant desdits premier (11a, 21a) et deuxième (11b, 21b) motifs d'écoulement dans ladite région de travail ; et
b) la zone sur laquelle la fonction de vitesse desdits premier (11a, 21a) et deuxième (11b, 21b) motifs d'écoulement varie de façon monotone dans ladite région de travail sont mutuellement augmentés à un maximum.

2. Appareil (10, 20) selon la revendication 1, comprenant en outre des moyens de commande pouvant être utilisés de sorte que ledit moyen d'alimentation et ledit moyen de retrait puissent être utilisés de manière sélective pour représenter alternativement lesdites première et deuxième paires de source-collecteur.

3. Appareil (10, 20) selon la revendication 1 ou 2, dans lequel lesdites première et deuxième paires de source-collecteur comprennent chacune une source ponctuelle et un collecteur ponctuel.

4. Appareil (20) selon la revendication 3, dans lequel ledit moyen d'alimentation comprend une ou plusieurs seringues (22a, 22b), pouvant être utilisées pour injecter une substance dans la chambre (25), et dans lequel ledit moyen de retrait comprend une ou plusieurs seringues (22a, 22b) pouvant être utilisées pour retirer une substance de la chambre (25).

5. Appareil (40) selon la revendication 1 ou 2, comprenant des premier et deuxième moyens d'alimentation et des premier et deuxième moyens de retrait, dans lequel chaque moyen d'alimentation comprend une source linéaire (46a, 46b) et chaque moyen de retrait comprend un collecteur linéaire (47a, 47b).

6. Appareil (40) selon la revendication 5, dans lequel ledit moyen d'alimentation comprend des moyens pour délivrer un fluide à la chambre (45) de sorte que le profil de vitesse du fluide varie de façon monotone à travers la région de travail de la chambre (45).

**7.** Appareil (10, 20) selon l'une quelconque des revendications précédentes, dans lequel la chambre (15, 25) a un périmètre avec une forme sensiblement circulaire.

**8.** Appareil (40) selon l'une quelconque des revendications 1 à 6, dans lequel la chambre (45) a un périmètre avec une forme sensiblement carrée ou rectangulaire.

**9.** Appareil (40) selon la revendication 8, lorsqu'elle est jointe à la revendication 5 ou 6, dans lequel lesdits premier et deuxième moyens d'alimentation sont prévus sensiblement le long de premier et deuxième bords orthogonaux adjacents de la chambre (45), et dans lequel chaque moyen de retrait est positionné le long du bord faisant face au moyen d'alimentation correspondant.

**10.** Appareil (10, 20, 40) selon l'une quelconque des revendications précédentes, dans lequel l'appareil comprend un microréseau ADN.

**11.** Appareil (10, 20) selon la revendication 1, dans lequel ledit moyen d'alimentation et ledit moyen de retrait consistent en trois noeuds qui peuvent être utilisés de manière à comprendre lesdites première et deuxième paires de source-collecteur, dans lequel un dit noeud est un noeud commun, lequel noeud commun comprend une source ou un collecteur de ladite première paire de source-collecteur et une source ou un collecteur de ladite deuxième paire de source-collecteur,

dans lequel lesdits noeuds sont positionnés de sorte que des première (24a) et deuxième (24b) lignes les plus courtes, lesquelles lignes les plus courtes (24a, 24b) représentent la distance la plus courte entre le noeud commun et chacun des autres noeuds, définissent mutuellement ledit angle de travail entre 60 et 120 degrés, dans lequel lesdits noeuds sont positionnés par rapport à la chambre (15, 25) de sorte que ledit angle de travail agisse sur la majeure partie de la chambre (15, 25), y compris la région centrale de celle-ci.

**12.** Appareil (10, 20) selon la revendication 1, dans lequel ledit moyen d'alimentation et ledit moyen de retrait consistent en trois noeuds qui peuvent être utilisés de manière à comprendre lesdites première et deuxième paires de source-collecteur, dans lequel un dit noeud est un noeud commun, lequel noeud commun comprend une source ou un collecteur de ladite première paire de source-collecteur et une source ou un collecteur de ladite deuxième paire de source-collecteur, dans lequel lesdits noeuds sont positionnés de sorte que des première (24a) et deuxième (24b) lignes les plus courtes, lesquelles lignes les plus courtes (24a, 24b) représentent la distance la plus courte entre le noeud commun et chacun des autres noeuds, définissent mutuellement ledit angle de travail entre 60 et 120 degrés, dans lequel lesdits noeuds sont tous positionnés dans, ou à proximité de, la région externe de la chambre (15, 25).

**13.** Appareil (40) selon la revendication 1, dans lequel ledit moyen d'alimentation et ledit moyen de retrait sont positionnés de sorte qu'une première ligne la plus courte entre la source de la première paire de source-collecteur et le collecteur correspondant soit sensiblement orthogonale à une deuxième ligne la plus courte entre la source de la deuxième paire de source-collecteur et le collecteur correspondant, lesdites première et deuxième lignes les plus courtes représentant la distance la plus courte entre une dite source et le collecteur correspondant, dans lequel ladite chambre (45) a une forme carrée ou rectangulaire et dans lequel lesdites première et deuxième lignes les plus courtes s'étendent sensiblement le long de bords orthogonaux adjacents de la chambre.

**14.** Appareil (10, 20) selon la revendication 1, dans lequel ledit moyen d'alimentation et ledit moyen de retrait sont configurés de sorte qu'une première ligne la plus courte (24a) entre la source de la première paire de source-collecteur et le collecteur correspondant définisse ledit angle de travail entre 60 et 120 degrés par rapport à une ligne la plus courte (24b) entre la source de la deuxième paire de source-collecteur et le collecteur correspondant, lesdites première et deuxième lignes les plus courtes (24a, 24b) représentant la distance la plus courte entre la source respective et le collecteur correspondant, dans lequel ladite chambre (15, 25) a une forme circulaire et dans lequel ledit moyen d'alimentation et ledit moyen de retrait sont configurés de sorte que le point d'intersection, ou le point d'intersection projeté, desdites lignes les plus courtes (24a, 24b) soit dans ou à proximité de la région externe de la chambre (15, 25), et de sorte que ledit angle de travail agisse sur la majeure partie de la chambre (15, 25), y compris la région centrale de celle-ci.

**15.** Procédé de conception d'un appareil (10, 20, 40) pour induire un mouvement de particules d'une ou de plusieurs substances, l'appareil (10, 20, 40) comprenant une chambre (15, 25, 45) comportant un moyen d'alimentation, pouvant être utilisé pour délivrer une substance à la chambre (15, 25, 45), et un moyen de retrait, pouvant être utilisé pour retirer une substance de la chambre (15, 25, 45), dans lequel ladite chambre (15, 25, 45) comprend une région de travail, ledit moyen d'alimentation et ledit moyen de retrait pouvant en outre être utilisés pour représenter des première et deuxième paires de source-collecteur, chaque paire de source-collecteur comprenant une source et un collecteur correspondant, et dans lequel le procédé comprend les étapes consistant à :

i) déterminer un premier motif d'écoulement (11a, 21a) qui représente le mouvement de particules se déplaçant de la source de ladite première paire de source-collecteur vers le collecteur correspondant de ladite première paire de source-collecteur, ledit premier motif d'écoulement (11a, 21a) comprenant une pluralité de lignes de courant ;

ii) déterminer un deuxième motif d'écoulement (11b, 21b) qui représente le mouvement de particules se déplaçant de la source de ladite deuxième paire de source-collecteur vers le collecteur correspondant de ladite deuxième paire de source-collecteur, ledit deuxième motif d'écoulement (11b, 21b) comprenant une pluralité de lignes de courant ;

iii) déterminer les emplacements desdites première et deuxième paires de source-collecteur dans ladite chambre (15, 25, 45) ;

**caractérisé en ce que** la ligne de courant (14a, 24a) du premier motif d'écoulement (11a, 21a) qui représente les particules se déplaçant avec la vitesse la plus grande définit un angle de travail entre 60 et 120 degrés avec la ligne de courant (14b, 24b) du deuxième motif d'écoulement (11b, 21b) qui représente les particules se déplaçant avec la vitesse la plus grande, et **en ce que** la ligne de courant (14a, 24a) du premier motif d'écoulement (11a, 21a) qui représente les particules se déplaçant avec la vitesse la plus grande ne croise pas la ligne de courant (14b, 24b) du deuxième motif d'écoulement (11b, 21b) qui représente les particules se déplaçant avec la vitesse la plus grande dans une région centrale de la région de travail, de sorte que :

a) le degré de transversalité du croisement des lignes de courant desdits premier (11a, 21a) et deuxième (11b, 21b) motifs d'écoulement dans ladite région de travail ; et

b) la zone sur laquelle la fonction de vitesse desdits premier (11a, 21a) et deuxième (11b, 21b) motifs d'écoulement varie de façon monotone dans ladite région de travail sont mutuellement augmentés à un maximum ;

iv) réaliser ledit appareil (10, 20, 40) de sorte que ledit moyen d'alimentation et ledit moyen de retrait soient positionnés dans ladite chambre (15, 25, 45) de manière à représenter les emplacements desdites première et deuxième paires de source-collecteur sélectionnés conformément à l'étape iii).

16. Programme d'ordinateur qui, lorsqu'il est chargé dans un ordinateur, amène l'ordinateur à effectuer le procédé de la revendication 15.

17. Programme d'ordinateur selon la revendication 16, supporté par un moyen de support.

18. Programme d'ordinateur selon la revendication 16, dans lequel ledit moyen de support est un moyen d'enregistrement.

19. Programme d'ordinateur selon la revendication 16, dans lequel ledit moyen de support est un moyen de transmission.

*Fig.1(A)*

*Fig.1(B)*

Fig.1(C)

Fig.1(D)

Fig.2(a)

T = 0.15

Fig.2(b)

T = 0.5

Fig.2(c)

T = 0.556

Fig.2(d)

T = 0.6

Fig.3(a)
T = 0.3

Fig.3(b)
T = 0.556

Fig.3(c)
T = 0.7

Fig.3(d)
T = 2.2

Fig.4(A)

Fig.4(B)

*Fig.5(A)*

*Fig.5(B)*

*Fig.6(a)*
T = 0.2

*Fig.6(b)*
T = 0.7

*Fig.6(c)*
T = 0.1

*Fig.6(d)*
T = 0.6

Fig.7(A)

Fig.7(B)

48

Reinjection

40

47a

46a

Line source 1

Line sink 1

45

*Fig.8(A)*

47b

Line sink 2

40

45

48

Reinjection

Line source 2

46b

*Fig.8(B)*

## Fig.9(A)

Horizontal source-sink pair

## Fig.9(B)

Vertical source-sink pair

Fig.10(a)
r = 1.8

Fig.10(b)
r = 3.5

Fig.10(c)
r = 4.0

Fig.10(d)
r = 4.3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **Evans J et al.** Planar Laminar Mixer. *MEMS. Proceedings IEEE of the Annual International Workshop of Micro Electromechanical Systems,* 26 January 1997
- Optimization of a pulsed source-sink microscale mixing device. Baratunde Aole Cola. Vanderbilt University, 01 December 2004
- **Jones, S. W. ; Aref, H.** Chaotic advection in pulsed source-sink systems. *Phys. Fluids,* vol. 31 (3), 469-485
- **Cola, B. A. ; Schaffer, D. K. ; Fisher, T. S. ; Stremler, M. A.** A pulsed source-sink fluid mixing device. *Journal of Microelectromechanical Systems,* 2006, vol. 15 (1), 259-265
- **McQuain, M. K. ; Seale, K. ; Peek, J. ; Fisher, T. S. ; Levy, S. ; Stremler, M. A. ; Haselton, F. R.** Chaotic mixer improves microarray hybridisation. *Analytical Biochemistry,* vol. 325, 215-226
- **Batchelor, G. K.** An Introduction to Fluid Dynamics. Cambridge University Press, 1967